(19) **Europäisches Patentamt / European Patent Office / Office européen des brevets**

(11) **EP 4 105 881 B1**

(12) **EUROPEAN PATENT SPECIFICATION**

(45) Date of publication and mention
of the grant of the patent:
**30.07.2025 Bulletin 2025/31**

(21) Application number: **21754469.1**

(22) Date of filing: **05.02.2021**

(51) International Patent Classification (IPC):
**G06T 7/00** *(2017.01)* **G01N 33/50** *(2006.01)*
**G06F 16/906** *(2019.01)* **G06N 20/00** *(2019.01)*
**G16B 40/20** *(2019.01)*

(52) Cooperative Patent Classification (CPC):
**G16B 40/20; G06F 18/214; G06F 18/285;**
**G06N 5/01; G06N 20/20; G06T 7/0012;**
**G06V 20/69;** G06T 2207/20081; G06T 2207/30024;
G06T 2207/30096

(86) International application number:
**PCT/JP2021/004193**

(87) International publication number:
**WO 2021/161901 (19.08.2021 Gazette 2021/33)**

(54) **FEATURE VALUE SELECTION METHOD, FEATURE VALUE SELECTION PROGRAM, MULTICLASS CLASSIFICATION METHOD, MULTICLASS CLASSIFICATION PROGRAM, FEATURE VALUE SELECTION DEVICE, MULTICLASS CLASSIFICATION DEVICE, AND FEATURE VALUE SET**

VERFAHREN ZUR AUSWAHL VON MERKMALWERTEN, PROGRAMM ZUR AUSWAHL VON MERKMALWERTEN, VERFAHREN ZUR KLASSIFIKATION MIT MEHREREN KLASSEN, PROGRAMM ZUR KLASSIFIKATION MIT MEHREREN KLASSEN, VORRICHTUNG ZUR AUSWAHL VON MERKMALSWERTEN, VORRICHTUNG ZUR KLASSIFIKATION MIT MEHREREN KLASSEN UND MERKMALSWERTSATZ

PROCÉDÉ DE SÉLECTION DE VALEUR DE CARACTÉRISTIQUE, PROGRAMME DE SÉLECTION DE VALEUR DE CARACTÉRISTIQUE, PROCÉDÉ DE CLASSIFICATION MULTICLASSE, PROGRAMME DE CLASSIFICATION MULTICLASSE, DISPOSITIF DE SÉLECTION DE VALEUR DE CARACTÉRISTIQUE, DISPOSITIF DE CLASSIFICATION MULTICLASSE ET ENSEMBLE DE VALEURS DE CARACTÉRISTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.02.2020 JP 2020022822**

(43) Date of publication of application:
**21.12.2022 Bulletin 2022/51**

(73) Proprietor: **FUJIFILM Corporation**
**Tokyo 106-8620 (JP)**

(72) Inventor: **NAGASE, Masaya**
**Ashigarakami-gun, Kanagawa 258-8538 (JP)**

(74) Representative: **Dehns Germany Partnerschaft mbB**
**Theresienstraße 6-8**
**80333 München (DE)**

(56) References cited:
**WO-A1-2008/139825     WO-A1-2012/111235**
**JP-A- 2011 181 016     JP-A- 2012 123 782**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- JI HYEON ET AL: "Feature selection for multi-class classification using pairwise class discriminatory measure and covering concept", ELECTRONICS LETTERS, THE INSTITUTION OF ENGINEERING AND TECHNOLOGY, GB, vol. 36, no. 6, 16 March 2000 (2000-03-16), pages 524 - 525, XP006015012, ISSN: 0013-5194, DOI: 10.1049/EL:20000458
- PO-WHEI HUANG ET AL: "Automatic Classification for Pathological Prostate Images Based on Fractal Analysis", IEEE TRANSACTIONS ON MEDICAL IMAGING, IEEE, USA, vol. 28, no. 7, 1 July 2009 (2009-07-01), pages 1037 - 1050, XP011250008, ISSN: 0278-0062
- HASTIE T ET AL: "Classification by pairwise coupling", ADVANCES IN NEURAL INFORMATION PROCESSING SYSTEMS 8 (NIPS 1995),, vol. 10, 1 January 1998 (1998-01-01), pages 1 - 38, XP002236002

**Description**

**BACKGROUND OF THE INVENTION**

1. Field of the Invention

The present invention relates to a multi-class classification method, a multi-class classification program, and a multi-class classification device which select a feature amount and classify a sample into any of a plurality of classes based on a value of the selected feature amount, and a feature amount selection method, a feature amount selection device, and a feature amount set which are used for such multi-class classification.

2. Description of the Related Art

In recent years, although the application or expansion of machine learning in the industrial field progresses, the feature selection and the multi-class classification are still major issues. There are various feature selection methods, but an example focusing on a pairwise coupling of the class is proposed (see "Feature selection for multi-class classification using pairwise class discriminatory measure and covering concept", Hyeon Ji et al., ELECTRONICS LETTERS, 16th March 2000, vol.36, No.6, p.524-525 below). The technology disclosed in "Feature selection for multi-class classification using pairwise class discriminatory measure and covering concept", Hyeon Ji et al., ELECTRONICS LETTERS, 16th March 2000, vol.36, No.6, p.524-525 is a method focusing on that the basic class classification is "binary-class classification" with two classes, using the pairwise coupling of the class to focus on the discrimination ability of the feature amount and perform the selection.

In addition, as a method of multi-class classification, for example, a one-versus-one (OVO) method in which two-class discrimination is repeated is known.

In addition, in the field of biotechnology, for example, methods of the feature selection and the multi-class classification are actively studied for cancer and the like. Generally, it is an application of a general machine learning method, and for example, a method of the feature selection by t-test, information gain, or the like, a classification method by support vector machine (SVM), random forest, naive bayes, or the like is applied. Such a technology is disclosed in JP2012-505453A, for example.

**SUMMARY OF THE INVENTION**

The study disclosed in "Feature selection for multi-class classification using pairwise class discriminatory measure and covering concept", Hyeon Ji et al., ELECTRONICS LETTERS, 16th March 2000, vol.36, No.6, p.524-525 is limited to only the feature selection, and uses the existing method as it is in the subsequent multi-class classification. In addition, the extension to a set cover problem, which will be described below for the present invention, is not specified. Also, independence between feature amounts for selecting robust feature amounts is verified, only basic multi-class classification is assumed, and discrimination unneeded classes are not introduced. Therefore, it is difficult to apply the study as it is to the extended multi-class classification. Similarly, in the technology disclosed in JP2012-505453A, it is not considered to examine a gene cluster needed for discrimination as the set cover problem.

In addition, in the methods of repeating the two-class discrimination and performing the multi-class classification, the problem that "higher ranking cannot be trusted" is pointed out in a voting method. In addition, in the tournament hierarchy method, the problem that it is difficult to decide a comparison order is pointed out.

In cases of the feature amount selection and the multi-class classification in the field of biotechnology, there is a problem that "the accuracy drops in a case in which the number of classes handled reaches about 10" in a case of mRNA expression level base, which is often reported. For example, in one of the reports that develops a multi-class cancer classifier based on mutation information, the result is that 5 types of cancer can be discriminated with an F-Number exceeding 0.70. The feature selection and the multi-class classification based on DNA methylation are also studied. However, the applied class remains in a small number of small sample-sized trials.

In recent years, although there is also the study that applies deep learning, in the first place, learning does not proceed well due to the undetermined problem of omics data (a sample size is small relative to the number of parameters; there are hundreds of thousands of methylated sites, whereas less than 10,000 open data tumor records are available). Even in a case in which study succeeds, there is a problem that, in diagnostic applications, it is difficult to accept the study because the reason for discrimination cannot be clarified.

As described above, in the related art, a sample having a plurality of feature amounts cannot be robustly and highly accurately classified into any of a plurality of classes based on a value of a part of the selected feature amount.

The present invention has been made in view of such circumstances, and is to provide a multi-class classification method, a multi-class classification program, and a multi-class classification device which can robustly and highly accurately classify a sample having a plurality of feature amounts into any of a plurality of classes based on a value of a part of the selected feature amount. In addition, the present invention is to provide a feature amount selection method, a feature amount selection device, and a feature amount set used for such multi-class classification.

A first aspect of the present invention relates to a feature amount selection method of selecting a feature amount group to be used for determining which of N (two or more) classes a sample belongs to according to claim 1. The method comprising an input step of inputting a learning data set including a known sample group belonging to a given class, which is a target, and a feature amount group of the known sample group, and a selection step of selecting a feature amount group needed for class determination for an unknown sample of which a belonging class is unknown, from the feature amount group based on the learning data set, in which the selection step includes a quantification step of quantifying, by a pairwise coupling that combines two classes among the N classes, a discrimination possibility between the two classes in accordance with each feature amount of the selected feature amount group by using the learning data set, and an optimization step of totalizing the quantified discrimination possibilities for all the pairwise couplings and selecting a combination of the feature amount groups for which a result of the totalization is optimized, a first marking step of marking a part of the given classes as first discrimination unneeded class groups that do not need to be discriminated from each other, and a first exclusion step of excluding the pairwise coupling in the marked first discrimination unneeded class groups from pairwise couplings to be expanded.

In an embodiment, the selection step further includes a similarity evaluation step of evaluating similarity between the feature amounts based on the discrimination possibility for each pairwise coupling of each feature amount, and a priority setting step of setting a priority of the feature amount to be selected, based on an evaluation result of the similarity.

In an embodiment, the similarity is an overlap relationship and/or an inclusion relationship of the discrimination possibility for each pairwise coupling.

In an embodiment, the similarity is a distance between discrimination possibility vectors for each pairwise coupling or a metric value in accordance with the distance.

An embodiment further comprises a selected number input step of inputting a selected number M of the feature amounts in the selection step, in which the optimization is maximizing a minimum value of a totalized value in all the pairwise couplings in accordance with M selected feature amounts.

In an embodiment, the optimization step includes an importance input step of inputting importance of the class or pairwise discrimination, and a weighting step of performing weighting based on the importance in a case of the totalization.

In an embodiment, the number of the feature amounts selected in the selection step is 25 or more.

In an embodiment, the number of the feature amounts selected in the selection step is 50 or more.

In an embodiment, the number of the feature amounts selected in the selection step is 100 or more.

Another aspect of the present invention relates to a feature amount selection program causing a computer to execute the feature amount selection method according to the first aspect.

In an embodiment, in a case in which N is an integer of 2 or more, which of N classes a sample belongs to, from a feature amount of the sample, the method comprising the input step and the selection step executed by using the feature amount selection method according to any one of the first aspect, and a determination step of performing the class determination for the unknown sample based on the selected feature amount group, which includes an acquisition step of acquiring a feature amount value of the selected feature amount group and a class determination step of performing the class determination based on the acquired feature amount value, in which, in the determination step, the class determination for the unknown sample is performed by configuring a multi-class discriminator that uses the selected feature amount group in association with the pairwise coupling.

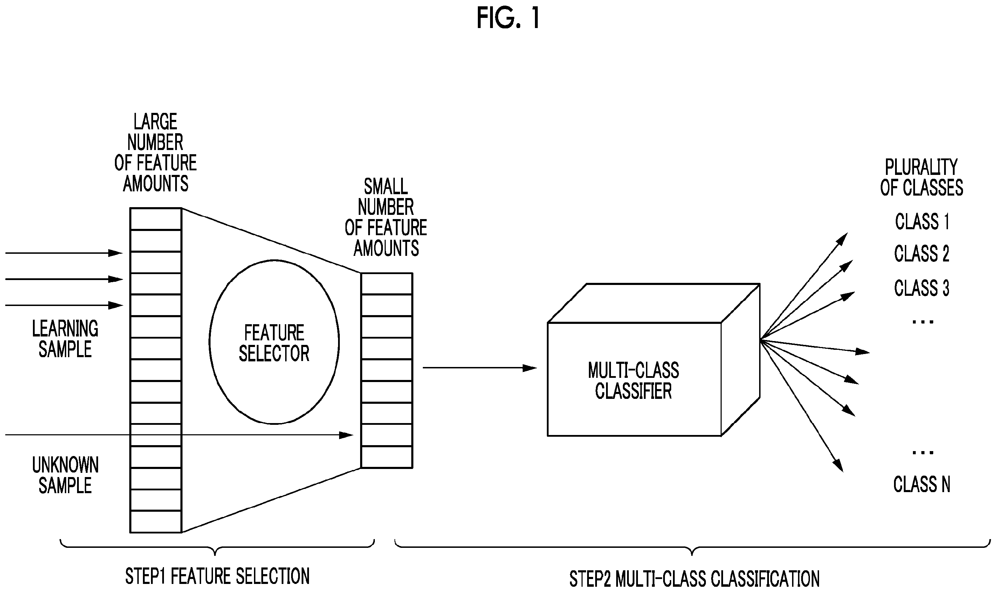

Fig. 1 is a schematic diagram of a multi-class classification problem involving feature amount selection, which is handled by the present invention. The feature selection (STEP 1) is a method (the feature amount selection method according to any one of the first aspect) of literally selecting in advance the feature amount needed for each subsequent processing (particularly, the multi-class classification in the present invention) from among a large number of the feature amounts included in the sample. That is, a large number of the feature amounts are acquired in advance in a certain data set (so-called learning data set), and the feature amounts (feature amount set) needed for each subsequent processing are selected based on the information. Then, in a case in which the (unknown) sample is actually given, only a small number of the feature amounts (feature amount set) selected in advance are referred to perform the multi-class classification. It should be noted that, since the unknown sample is classified in accordance with the feature amount selected only in the learning data set, it is naturally desirable that the feature amount is robust.

The feature selection is particularly useful in a case in which it takes cost (including time, cost, and the like) to refer to (including acquisition, storage, and the like) the feature amount of the sample. Therefore, for example, a unit that refers to the feature amount of the learning data and a unit that refers to the feature amount of the unknown sample may be different, and after selecting a small number of feature amounts, a suitable feature amount acquisition unit may be developed and prepared.

On the other hand, the multi-class classification (STEP2) is a discrimination problem of deciding which of a plurality of classes the given unknown sample belongs to, and is a general problem in machine learning. It should be noted that many of the actual multi-class classifications are not always the problem of simply selecting one of the N classes. For example, even in a case in which the plurality of classes actually are present, the discrimination itself may not be needed. Conversely, for example, in a sample set labeled as one class, a plurality of sample groups having different appearances may be mixed. The method withstands such a complicated extended multi-class classification is desirable.

As the simplest feature selection method, it is conceivable to evaluate all selection methods of a small number of the feature amounts from a large number of the feature amounts, which are candidates, by using the learning data set. However, since there is a risk of over-learning for the learning data set, and the number of candidates is huge and cannot be evaluated, some kind of framework is essential.

An example of applying the first aspect of the present invention (multi-class classification involving the feature selection) to the field of biotechnology is shown. Cancer or a body tissue has a unique DNA methylated pattern. In addition, DNA liberated from the body tissue (cell free DNA: cfDNA) is mixed in human blood, and in particular, cfDNA derived from cancer is detected. Therefore, by analyzing the methylated pattern of cfDNA, it is possible to determine the presence or absence of the cancer and to specify the primary lesion in a case in which the cancer is present. That is, early cancer screening test by blood sampling and guidance to appropriate detailed test are realized.

Therefore, the problem of discriminating "whether it is cancer or non-cancer" and the origin tissue from the DNA methylated pattern is extremely important. This problem can be defined as the multi-class classification problem that discriminates the cancer from blood or a normal tissue. However, since there are many types of human organs (for example, 8 types of major cancers and 20 types or more of normal tissues), and there are subtypes of cancer, so that some cancers of the same organ have different aspects from each other, it can be said that the classification problem is difficult.

In addition, suppressing the measurement cost is desired from the assumption that it is used for the screening test, so that an expensive array that comprehensively measures methylated sites cannot be used as it is. Therefore, it is necessary to narrow down in advance a small number of sites needed for the discrimination from hundreds of thousands of DNA methylated sites, that is, the feature selection is needed in the previous stage.

Therefore, the technology (the method proposed in the present invention) of narrowing down a small number of DNA methylated sites and configuring the feature selection and multi-class classification methods that can discriminate the cancer from the normal tissue and specify the origin tissue based on the small number of sites is useful. It should be noted that, since the number of DNA methylated sites selected from, for example, 300,000 sites exceeds 10 to the 1,000th power, it can be seen that a comprehensive search method cannot be used.

Therefore, the inventors of the present application propose the feature selection method of listing the DNA methylated sites that act like switches that contribute to robust discrimination and being based on the combination search that sufficiently covers the pairwise discrimination of the needed classes. Further, the inventors of the present application propose the method of configuring a multi-class classifier from a simple binary-class classifier in combination with a tournament hierarchy method by only using a robust discrimination portion among the selected sites.

As a result, it is possible to support the multi-class classification involving the feature selection that incorporates various characteristics of actual problems. Actually, it can be applied to the multi-class classification that greatly exceeds 10 classes of cancer and normal, as seen in the example of cancer diagnosis described above. The feature amount selection and the multi-class classification methods proposed by the inventors of the present application are extremely useful in industry.

It should be noted that the present description is one of specific cases, and the the present invention is not applicable only to the field of biotechnology. Actually, as many of the common machine learning technologies are applicable to the field of biotechnology, there is no problem even in a case in which the technology developed in the field of biotechnology is applied to general machine learning problems.

In an embodiment, the quantification step, a statistically significant difference in the feature amounts in the learning data set between pairwise-coupled classes is used.

In an embodiment, the quantification step, in a case in which a feature amount of the unknown sample belonging to any of pairwise-coupled classes is given under a threshold value set with reference to the learning data set, a probability of correctly discriminating a class to which the unknown sample belongs by the given feature amount is used.

In an embodiment, the quantification step, a quantification value of the discrimination possibility is a value obtained by performing multiple test correction on a statistical probability value by the number of feature amounts.

An embodiment further comprises a subclass setting step of clustering one or more samples belonging to the classes based on a given feature amount from the learning data set to form a cluster and setting the formed cluster to a subclass in each class, a second marking step of marking each subclass in each class as second discrimination unneeded class groups that do not need to be discriminated from each other in each class, and a second exclusion step of excluding the pairwise coupling in the marked second discrimination unneeded class groups from pairwise couplings to be expanded.

In an embodiment, the totalization is calculating a total value or an average value of quantitative values of the discrimination possibility.

An embodiment further comprises a target threshold value input step of inputting a target threshold value T of a totalized value indicating a result of the totalization, in which the optimization is setting a minimum value of the totalized value in all the pairwise couplings in accordance with a selected feature amount to be equal to or more than the target threshold value T.

In an embodiment, the determination step, binary-class discriminators that each use the selected feature amount group in association with each pairwise coupling are configured, and the binary-class discriminators are combined to configure the multi-class discriminator.

An embodiment further comprises a step of evaluating a degree of similarity between the sample and each class by a binary-class discriminator, and a step of configuring the multi-class discriminator based on the degree of similarity.

An embodiment further comprises a step of evaluating a degree of similarity between the sample and each class by a binary-class discriminator, and a step of configuring the multi-class discriminator by applying the binary-class discriminator used for evaluation of the degree of similarity between classes having a higher degree of similarity again to the classes.

In an embodiment, the determination step, a decision tree that uses the selected feature amount group in association with each pairwise coupling is configured, and one or more decision trees are combined to configure the multi-class discriminator.

In an embodiment, the determination step, the multi-class discriminator is configured by a combination of the decision tree and the decision tree, as a random forest.

In an embodiment, omics information of a living body tissue piece is measured to determine a class to which the living body tissue piece belongs from the N classes.

In an embodiment, omics switch-like information of a living body tissue piece is measured to determine a class to which the living body tissue piece belongs from the N classes.

In an embodiment, the number of classes to be discriminated is 10 or more.

In an embodiment, the number of the classes to be discriminated is 25 or more.

A further aspect of the present invention relates to a multi-class classification program causing a computer to execute the multi-class classification method as defined above. It should be noted that a non-transitory recording medium on which a computer-readable code of the program according to the twenty-eighth aspect is recorded can also be used as an aspect of the present invention.

A further aspect of the present invention relates to a feature amount selection device that selects a feature amount group to be used for determining which of N (two or more) classes a sample belongs to according to claim 12, the device comprising a first processor, in which the first processor performs input processing of inputting a learning data set including a known sample group belonging to a given class, which is a target, and a feature amount group of the known sample group, and selection processing of selecting a feature amount group needed for class determination for an unknown sample of which a belonging class is unknown, from the feature amount group based on the learning data set, and the selection processing includes quantification processing of quantifying, by a pairwise coupling that combines two classes among the N classes, a discrimination possibility between the two classes in accordance with each feature amount of the selected feature amount group by using the learning data set, and optimization processing of totalizing the quantified discrimination possibilities for all the pairwise couplings and selecting a combination of the feature amount groups for which a result of the totalization is optimized, first marking processing of marking a part of the given classes as first discrimination unneeded class groups that do not need to be discriminated from each other, and first exclusion processing of excluding the pairwise coupling in the marked first discrimination unneeded class groups from pairwise couplings to be expanded.

In an embodiment, in a case in which N is an integer of 2 or more, which of N classes a sample belongs to, from a feature amount of the sample, the device comprising the feature amount selection device according to the twenty-ninth aspect, and a second processor, in which the second processor performs the input processing and the selection processing using the feature amount selection device, and determination processing of performing the class determination for the unknown sample based on the selected feature amount group, which includes acquisition processing of acquiring a feature amount value of the selected feature amount group and class determination processing of performing the class determination based on the acquired feature amount value, and in the determination processing, the class determination for the unknown sample is performed by configuring a multi-class discriminator that uses the selected feature amount group in association with the pairwise coupling.

A further aspect of the present invention relates to a feature amount set that is used by a multi-class classification device to determine which of N (two or more) classes a sample belongs to according to claim 15, the feature amount set comprising a feature amount data set of the sample belonging to each class, which is a target, in which, in a case in which, by a pairwise coupling that combines two classes among the N classes, a discrimination possibility between the two classes in accordance with each feature amount of the selected feature amount group is quantified with reference to the feature amount data set, the feature amount set is marked to be discriminable by at least one feature amount in all the pairwise couplings.

In an embodiment, in a case in which, by the pairwise coupling that combines the two classes among the N classes, the discrimination possibility between the two classes in accordance with each feature amount of the selected feature amount group is quantified with reference to the feature amount data set, the feature amount set is marked to be discriminable by at least 5 or more feature amounts in all the pairwise couplings.

**In** an embodiment, in a case in which, by the pairwise coupling that combines the two classes among the N classes, the discrimination possibility between the two classes in accordance with each feature amount of the selected feature amount group is quantified with reference to the feature amount data set, the feature amount set is marked to be discriminable by at least 10 or more feature amounts in all the pairwise couplings.

**In** an embodiment, in a case in which, by the pairwise coupling that combines the two classes among the N classes, the discrimination possibility between the two classes in accordance with each feature amount of the selected feature amount group is quantified with reference to the feature amount data set, the feature amount set is marked to be discriminable by at least 60 or more feature amounts in all the pairwise couplings.

In an embodiment, in which the number of selected feature amounts is equal to or less than 5 times a presented minimum cover number.

In an embodiment, the number of classes to be discriminated is 10 or more.

In an embodiment, the number of classes to be discriminated is 25 or more.

In an embodiment, the number of selected feature amounts is 25 or more.

In an embodiment, the number of the selected feature amounts is 50 or more.

In an embodiment, the number of the selected feature amounts is 100 or more.

## BRIEF DESCRIPTION OF THE DRAWINGS

Fig. 1 is a schematic diagram showing a multi-class classification problem involving feature amount selection.

Fig. 2 is a diagram showing a configuration of a multi-class classification device.

Fig. 3 is a diagram showing a configuration of a processing unit.

Fig. 4 is a flowchart showing processing of a multi-class classification method.

Figs. 5A and 5B are diagrams showing aspects of classification by a switch-like feature amount.

Figs. 6A to 6C are diagrams showing matrices of a discrimination switch value.

Figs. 7A and 7B are diagrams showing aspects of decision of a discrimination switch value and a state value.

Fig. 8 is a diagram showing exclusion of pairwise expansion between discrimination unneeded classes.

Figs. 9A and 9B are diagrams showing aspects of subclass introduction.

Figs. 10A to 10C are diagrams showing aspects of creating a brute force ranking.

Fig. 11 is a diagram showing an aspect of a final tournament match.

Fig. 12 is a diagram showing a detailed breakdown of a data set.

Fig. 13 is a diagram showing a comparison result of discrimination accuracy between the present invention and the related-art method.

Fig. 14 is a diagram showing a comparison result of robustness between the present invention and the related-art method.

Fig. 15 is a diagram showing a relationship between the number of selected feature amounts and the discrimination accuracy (F-number).

Fig. 16 is a table showing a shown example of a basis for the discrimination.

Fig. 17 is a diagram showing a relationship between the number of the selected feature amounts and a minimum cover number.

Fig. 18 is a table showing a relationship between the minimum cover number and a minimum F-number.

## DESCRIPTION OF THE PREFERRED EMBODIMENTS

In the following, embodiments of a feature amount selection method, a feature amount selection program, a multi-class classification method, a multi-class classification program, a feature amount selection device, a multi-class classification device, and a feature amount set according to the present invention will be described with reference to the attached drawings.

<First Embodiment>

<Schematic Configuration of Multi-Class Classification Device>

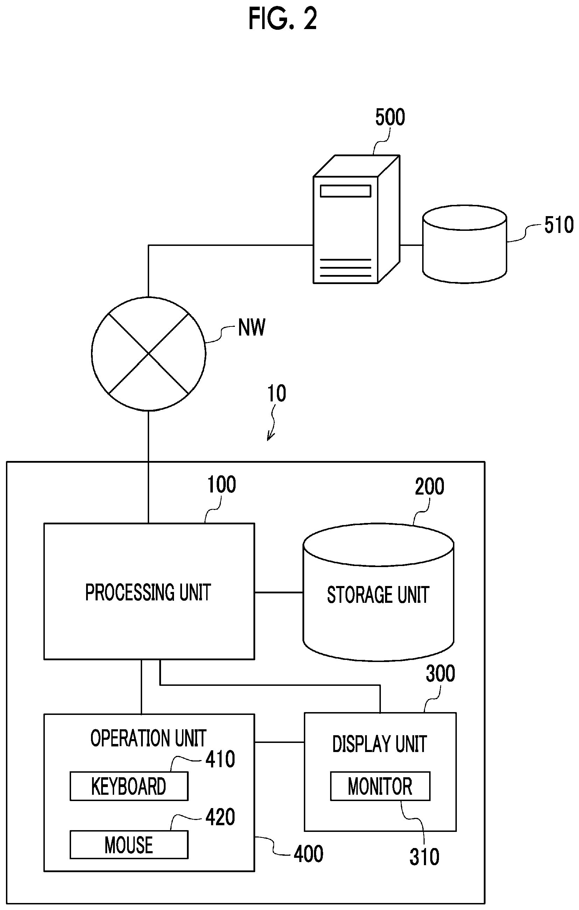

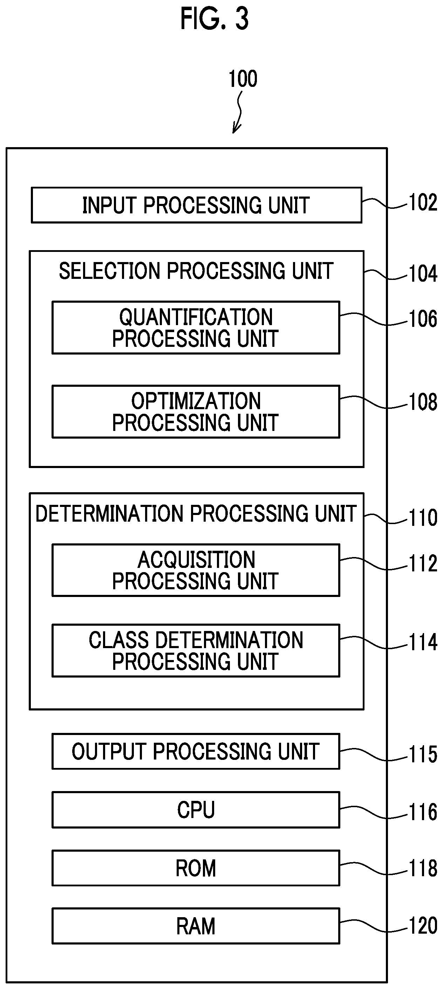

Fig. 2 is a diagram showing a schematic configuration of a multi-class classification device according to a first embodiment. As shown in Fig. 2, a multi-class classification device 10 (feature amount selection device and multi-class classification device) according to the first embodiment comprises a processing unit 100 (first processor and second processor), a storage unit 200, a display unit 300, and an operation unit 400, which are connected to each other to transmit and receive needed information. These constituent elements can be installed by employing various installation forms. The constituent elements may be installed in one site (in one housing, one room, or the like) or may be installed in places separated from each other and connected via a network. In addition, the multi-class classification device 10 (input processing unit 102; see Fig. 3) connects to an external server 500 and an external database 510 via a network NW, such as the Internet, and can acquire information, such as a sample for multi-class classification, a learning data set, and a feature amount set, as needed.

<Configuration of Processing Unit>

As shown in Fig. 3, the processing unit 100 comprises the input processing unit 102, a selection processing unit 104, a determination processing unit 110, a central processing unit (CPU) 116, a read only memory (ROM) 118, and a random access memory (RAM) 120. The input processing unit 102 inputs a learning data set including a known sample group of which a belonging class is known and a feature amount group of the known sample group from the storage unit 200 or a storage device on the network and performs input processing. The selection processing unit 104 performs selection processing of selecting a feature amount group needed for the class determination for an unknown sample of which a belonging class is unknown from the feature amount group based on the input learning data set, and comprises a quantification processing unit 106 and an optimization processing unit 108. The determination processing unit 110 performs class determination (determination processing) of the unknown sample based on the selected feature amount group, and comprises an acquisition processing unit 112 and a class determination processing unit 114. An output processing unit 115 outputs a processing condition or a processing result by displaying, storing, printing, or the like. It should be noted that the processing by each of these units is performed under the control of the CPU 116 (first processor and second processor).

The functions of the units of the processing unit 100 can be realized using various processors and a recording medium. The various processors include, for example, a central processing unit (CPU) which is a general-purpose processor which executes software (program) to realize various functions. In addition, the various processors also include a graphics processing unit (GPU) which is a processor specialized for image processing, and a programmable logic device (PLD) which is a processor of which a circuit configuration can be changed after manufacturing, such as a field programmable gate array (FPGA). In a case in which learning or recognition of the image is performed, the configuration using the GPU is effective. Further, the various processors also include a dedicated electric circuit which is a processor having a circuit configuration designed exclusively for executing specific processing, such as an application specific integrated circuit (ASIC).

The functions of the units may be realized by one processor, or may be realized by a plurality of processors of the same type or different types (for example, a plurality of FPGAs, or a combination of the CPU and the FPGA, or a combination of the CPU and the GPU). In addition, a plurality of the functions may be realized by one processor. As an example of configuring the plurality of functions with one processor, first, as represented by a computer, there is a form in which one processor is configured by a combination of one or more CPUs and software, and the processor realizes the plurality of functions. Second, as represented by a system-on-chip (SoC) or the like, there is a form in which a processor that realizes the functions of the entire system with one integrated circuit (IC) chip is used. As described above, various functions are configured by one or more of the various processors as the hardware structure. Further, the hardware structure of these various processors is more specifically an electric circuit (circuitry) in which circuit elements, such as semiconductor elements, are combined. The electric circuit may be an electric circuit that realizes the functions using a logical sum, a logical product, a logical negation, an exclusive logical sum, and a logical operation of a combination thereof.

In a case in which the processor or the electric circuit executes software (program), a code readable by a computer (for example, various processors or electric circuits constituting the processing unit 100 and/or a combination thereof) of the executed software is stored in a non-transitory recording medium, such as the ROM 118, and the computer refers to the software. The software stored in the non-transitory recording medium includes a program (feature amount selection program and multi-class classification program) for executing the feature amount selection method and/or the multi-class classification method according to the embodiment of the present invention, and data (data related to acquisition of the learning data, and data used for the feature amount selection and the class determination) used in a case of execution. The code may be recorded in the non-transitory recording medium, such as various magneto-optical recording device and a semiconductor memory, instead of the ROM 118. In a case of the processing using the software, for example, the RAM 120 is used as a transitory storage region, and the data stored in, for example, an electronically erasable and programmable read only memory (EEPROM) (not shown) can also be referred to. The storage unit 200 may be used as the "non-transitory recording medium".

Details of the processing by the processing unit 100 having the configuration described above will be described below.

<Configuration of Storage Unit>

The storage unit 200 is configured by various storage devices, such as a hard disk and a semiconductor memory, and a control unit thereof, and can store the learning data set described above, an execution condition of the selection processing and the class determination processing and results thereof, the feature amount set and the like. The feature amount set is the feature amount set that is used by the multi-class classification device 10 to determine which of N (two or more) (N is integer of 2 or more) classes the sample belongs to and comprises the feature amount data set of the sample belonging to each class, which is a target, in which, in a case in which, by a pairwise coupling that combines two classes among the N classes, a discrimination possibility between the two classes in accordance with each feature amount of the selected feature amount group is quantified with reference to the feature amount data set, the feature amount set is marked to be discriminable by at least one feature amount in all the pairwise couplings. The feature amount set can be generated by an input step (input processing) and a selection step (selection processing) in the feature amount selection method (feature amount selection device) according to the embodiment of the present invention. In addition, the feature amount set is preferably marked as discriminable with at least 5 or more feature amounts, more preferably marked as discriminable with at least 10 or more feature amounts, and still more preferably marked as discriminable with at least 60 or more feature amounts. In addition, the feature amount set is effective in a case in which the number of classes to be discriminated is 10 or more, and further effective in a case in which the number of classes is 25 or more. In addition, it is effective in a case in which the number of the selected feature amounts is 50 or more, and further effective in a case in which the number of the selected feature amounts is 100 or more.

<Configuration of Display Unit>

The display unit 300 comprises a monitor 310 (display device) configured by a display, such as a liquid crystal display, and can display the acquired learning data and the result of the selection processing and/or the class determination processing. The monitor 310 may be configured by a touch panel type display, and may receive command input by a user.

<Configuration of Operation Unit>

The operation unit 400 comprises a keyboard 410 and a mouse 420, and the user can perform operations related to execution of the multi-class classification method according to the embodiment of the present invention, result display, and the like via the operation unit 400.

<1. Processing of Feature Amount Selection Method and Multi-Class Classification Method>

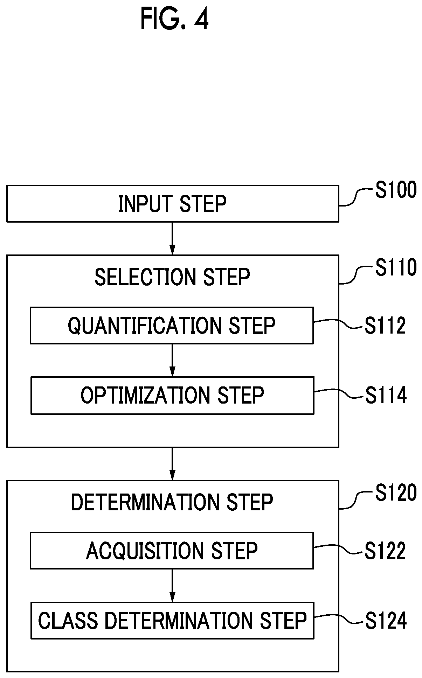

Fig. 4 is a flowchart showing basic processing of the feature amount selection method and the multi-class classification method according to the embodiment of the present invention. The feature amount selection method according to the embodiment of the present invention is the feature amount selection method of selecting the feature amount group used for determining which of N (two or more) classes the sample belongs to. In addition, the multi-class classification method according to the embodiment of the present invention is the multi-class classification method of determining, in a case in which N is an integer of 2 or more, which of N classes the sample belongs to, from the feature amount of the sample, the method including the input step (step S100) of inputting the learning data set including the known sample group belonging to a given class, which is a target, and the feature amount group of the known sample group, the selection step (step S110) of selecting the feature amount group needed for the class determination for the unknown sample of which the belonging class is unknown from the feature amount group based on the learning data set, and a determination step (step S120) of performing the class determination for the unknown sample based on the selected feature amount group, which includes an acquisition step (step S122) of acquiring a feature amount value of the selected feature amount group and a class determination step (step S124) of performing the class determination based on the acquired feature amount value.

The selection step includes a quantification step (step S112) of quantifying, by a pairwise coupling that combines two classes among the N classes, the discrimination possibility between the two classes in accordance with each feature amount of the selected feature amount group by using the learning data set, and an optimization step (step S114) of totalizing the quantified discrimination possibilities for all the pairwise couplings and selecting a combination of the feature amount groups for which a result of the totalization is optimized. In addition, in the determination step, the class determination for the unknown sample is performed by configuring the multi-class discriminator that uses the selected feature amount group in association with the pairwise coupling.

<2. Basic Policy of Present Invention>

The present invention is particularly suitable in a case of selecting the feature amount having a characteristic close to a binary value, and in a case of deciding the class by combining such feature amounts like a "switch". That is, it is not a case in which it is quantitatively bonded to the feature amount linearly or non-linearly, but this is not always simple, and it is a sufficiently complicated problem in a case in which there are many switches. Therefore, the present invention is based on the policy of "searching for and selecting a combination of a large number of the feature amounts having a switch-like function, and configuring the multi-class classifier with a simple classifier".

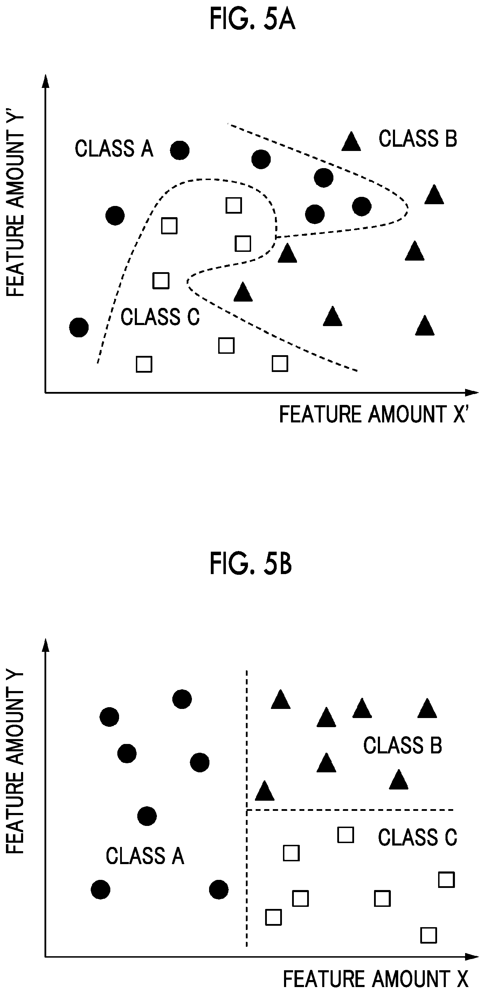

Figs. 5A and 5B are diagrams showing the "feature amount having the switch-like function" described above. Fig. 5A shows an aspect of the class classification based on a feature amount X' and the feature amount Y', which is complicated and non-linear classification. Fig. 5B shows an aspect of the class classification based on a feature amount X and a feature amount Y, which is simple and linear classification. From the viewpoint of high-accuracy and high-robust class classification, it is preferable to select the feature amount having the switch-like function as shown in Fig. 5B.

It should be noted that the learning data set is given, for every sample, values of a plurality of common feature amounts (for example, methylated sites) (note that some "missing values" may be included as values: hereinafter, referred to NA) and one correct answer class label (for example, cancer or non-cancer, and tissue classification) are given (input (input step and input processing: step S100) of the learning data set by the input processing unit 102 is performed).

In addition, although the above assumption is made here for the sake of simplicity, in a case in which a part of the sample is not given a correct answer class label, so-called semi-supervised learning may be incorporated. Since it is a combination with a known method, two typical processing examples are simply shown. A method (1) of, as a preprocessing, giving some class labels to the sample to which the correct answer class label is not given based on data comparison with the sample to which the correct answer class label is given, and a method (2) of performing cycle, such as estimating the belonging class of another unknown sample after learning with the data to which the class label is given once, and regarding the class label having high accuracy as the "correct label", increasing the learning data again, and performing learning can be used in combination.

<2.1 Feature Amount Selection Method>

In the present chapter, the selection of the feature amount (step S110: selection step) by the selection processing unit 104 (quantification processing unit 106 and optimization processing unit 108) will be described. First, the principle of feature amount selection (selection step, selection processing) according to the embodiment of the present invention will be described in a simplified case. In the following, a method of sequential extension will be described. Finally, the procedure of the feature amount selection that incorporates all the extensions is summarized. It should be noted that, understandably, all the feature amounts described in the present chapter refer to the learning data.

<2.2 Principle of Feature Amount Selection: Return to Set Cover Problem>

First, the principle of the feature amount selection (selection step) for the multi-class classification will be described. For the sake of simplicity in the present section, it is assumed that the values of all the feature amounts of the samples belonging to the same class exactly match, and the feature amounts take a fixed value of binary (0 or 1).

In a case in which a value of a feature amount i of a class s is denoted by $X_i^{(s)}$, "the classes s and t can be discriminated by the selected feature set f" means that any feature amount is different, that is, Expression (1) is satisfied.

$$\exists i \in f, X_i^{(s)} \neq X_i^{(t)} \quad \ldots \quad (1)$$

Therefore, a necessary and sufficient condition that all the given classes C = {1, 2, ..., N} can be discriminated from each other is that Expression (2) is satisfied.

$$\forall \{s, t \mid s \neq t \in C\}, \exists i \in f, X_i^{(s)} \neq X_i^{(t)} \quad \ldots \quad (2)$$

Here, a class binary relationship is pairwise expanded, the exclusive logical sum $Y_i^{(k)}$ (see Expression (3)) of the binary feature amounts i of the classes s and t is introduced for the pair k = {s, t} $\in P_2(C)$ in the binary combination, and this is called a "discrimination switch" (Figs. 5A and 5B).

$$Y_i^{(k=\{s,t\})} = \text{xor}\left(X_i^{(s)}, X_i^{(t)}\right) \quad \ldots \quad (3)$$

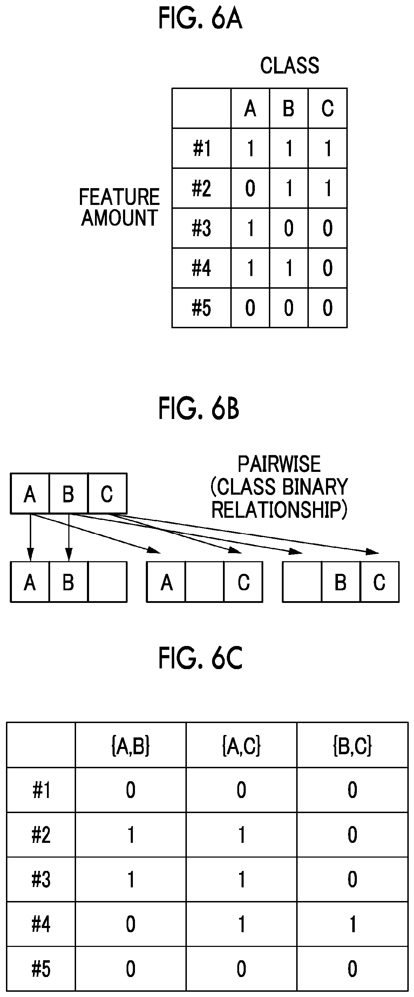

Figs. 6A to 6C are diagrams showing an aspect of calculation of a discrimination switch. Fig. 6A is a table showing the values of binary feature amounts #1 to #5 (values are 0 or 1; binary feature amount values) for classes A, B, and C, and Fig. 6B shows the aspect in which the classes A, B, and C are pairwise expanded to form pairs {A, B}, {A, C}, {B, C}. Fig. 6C shows the exclusive logical sum (value is 0 or 1; discrimination switch value) of the binary feature amounts for each pair. For example, the discrimination switch value of the feature amount #1 is 0 for the pair {A, B}, which means that "the pair {A, B} cannot be discriminated by the feature amount #1 (it cannot discriminate which of class A or B the sample belongs to)". On the other hand, for example, since the discrimination switch value of the feature amount #2 is 1 for the pair {A, B}, it can be seen that "the pair {A, B} can be discriminated by the value of the feature amount #2".

From the above, the necessary and sufficient condition that all the given classes C can be discriminated from each other can be rewritten as Expression (4).

$$\forall k \in P_2(C), \exists i \in f, Y_i^{(k)} = 1 \quad \ldots \quad (4)$$

That is, in a case in which a whole feature set is denoted by F, the feature amount selection for the multi-class classification can be returned to the set cover problem of selecting a subset f $\subseteq$ F satisfying Expression (4).

It should be noted that the "set cover problem" can be defined as, for example, "a problem of selecting a subset of S such that it includes (covers) all the elements of U at least once in a case in which the set U and the subset S of a power set of U are given" (other definitions are possible).

Here, the switch set $I_i = \{k | Y_i^{(k)} = 1\}$ for the feature amount i is the subset of the binary combination $P_2(C)$ of the class. Therefore, I = {Ii|i $\in$ F} corresponding to the whole feature set F is the subset of the power set of its family of sets, $P_2(C)$. That is, the problem is "a problem of selecting the subset (corresponding to f) of I such that it includes all elements of $P_2(C)$ at least once in a case in which the subset I (corresponding to F) of the power set of $P_2(C)$ is given", that is, it can be regarded as the set cover problem. Specifically, it is necessary to select the feature amount (and/or a combination thereof) such that at least one discrimination switch value is "1" for all pairs that are pairwise expanded. In the cases of Figs. 6A to 6C, "feature amounts #2 and #4", "feature amounts #3 and #4", or "feature amounts #2, #3, and #4" need only be selected. It should be noted that, in the value of the feature amount is NA, the pairwise discrimination switch value is automatically set to zero.

<2.3 Substitute Exclusive Logical Sum with Quantitative Value of Discrimination Possibility>

Here, in a case in which the feature amount is originally the binary value, the feature amount and its representative value (median value or the like) may be regarded as the discrimination possibility as it is. It should be noted that, in general, the feature amount is not limited to the binary value, and even samples belonging to the same class can fluctuate to various values. Therefore, the quantification processing unit 106 (selection processing unit 104) desirably substitutes the discrimination switch value (exclusive logical sum) with the quantitative value (quantification value) of discrimination possibility based on the feature amount of the learning data set.

First, the quantification processing unit 106 estimates the distribution parameter $\theta_i(s)$ and the distribution $D(\theta_i(s))$ of the class s and the feature amount i from the measurement value group of the feature amount i of the sample belonging to the class s (step S112: quantification step). It is particularly desirable to quantify the discrimination possibility from the distribution or the distribution parameter. It should be noted that, the sample of which the value of the feature amount is NA need only be excluded from the quantitative processing. Of course, in a case in which all the samples are NA, the feature amounts cannot be used.

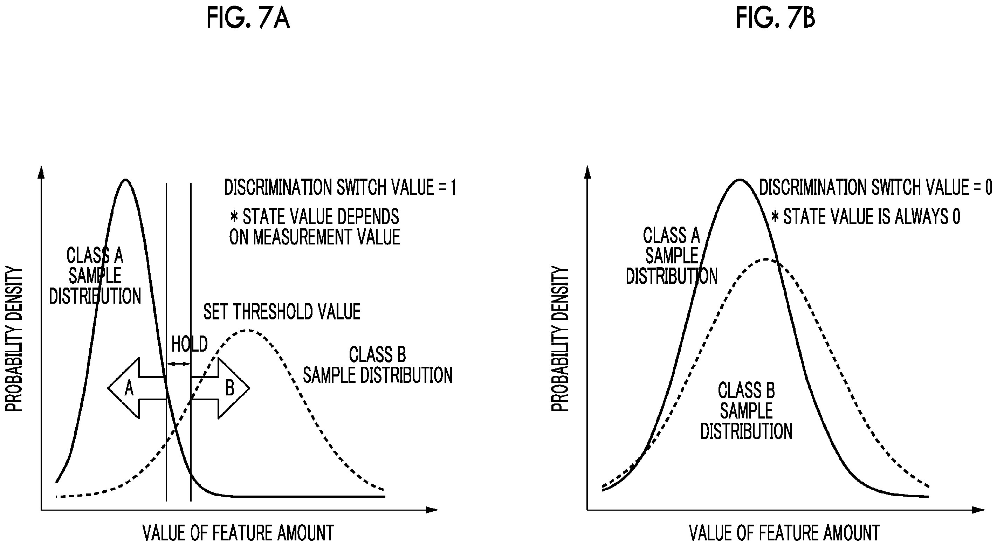

For example, the quantification processing unit 106 can obtain a p-value by performing a statistical test on the presence or absence of the significant difference between the pairwise parameter $\theta_i(s)$ and $\theta_i(t)$, specifically, can use Welch's t-test. The Welch's t-test is a method that assumes a normal distribution and is a general-purpose applicable method (as an image, the significant difference is determined depending on whether the feature amount distribution of s and t is close to any of Fig. 7A or Fig. 7B). Of course, based on the statistical properties of the feature amount, an observation result, or an analysis result, a timely and appropriate distribution and the corresponding statistical test method may be adopted.

Figs. 7A and 7B are diagrams showing decision images of the discrimination switch value and a state value. Fig. 7A is a case in which the feature amount is used for the discrimination of the pairwise {A, B}, and the quantification processing unit 106 sets a threshold value (value at a position of two vertical lines in Fig. 7A) from the learning data, and decides the discrimination switch state value from the measurement value of the target sample (step S112: quantification step). The state value is +1 in a case in which the measurement value belongs to an A side of the distribution, the state value is -1 in a case in which the measurement value belongs to a B side thereof, and the state value is 0 in a case in which the measurement value belongs to a holding region. Fig. 7B is a case in which the feature amount is not used for the discrimination of the pairwise {A, B} in the first place ($Y_i^{(\{A,B\})} = 0$).

It should be noted that, in a case in which there are a particularly large number of feature amount candidates, in a case in which the determination is repeated for whole feature set F, a multiple comparison test will occur. Therefore, the quantification processing unit 106 desirably corrects a p-value group obtained for the same pairwise k = {s, t} to a so-called q-value group (step S112: quantification step). Examples of the method of multiple test correction include the Bonferroni method and the BH method [Benjamini, Y., and Y. Hochberg, 1995], and more desirable method is the latter method of performing correction to a so-called false discovery rate (FDR), but it is not limited to this.

As shown in Expression (5), the quantification processing unit 106 compares the obtained q-value with a predetermined reference value $\alpha$ and assigns 0 or 1 to the discrimination switch (particularly, a case in which the discrimination switch is 1 is called "marked").

$$Y_i^{(k=\{s,t\})} = \begin{cases} 1 \text{ if } q \le \alpha \\ 0 \text{ if } q > \alpha \end{cases} \quad \cdots \quad (5)$$

It should be noted that, the discrimination switch is discretized and binarized from the standpoint of extending the set cover problem, but continuous variables may be handled, for example, by setting the discrimination switch to 1-q.

Further, since the p-value or the q-value is the statistical difference and not the probability that the sample can be discriminated, the quantification processing unit 106 may further perform, under the appropriate threshold value which is set with reference to the learning data set, the quantification by the probability that the belonging class can be correctly discriminated by the feature amount in a case in which the feature amount is given to the unknown sample belonging to any of the pairwise-coupled classes. In addition, the quantification processing unit 106 may correct such a statistical probability value by the multiple test correction in accordance with the number of the feature amounts.

In addition, in addition to the reference related to the statistical test, the reference value, such as having a certain difference in the average value, may be added or used as a substitute. Of course, various statistics other than the average value and the standard deviation may be used as the reference.

<2.4 Extension of Set Cover Problem to Optimization Problem, such as Maximizing Minimum Pairwise Cover Number>

In a case in which the feature amount is a random variable, even in a case in which the discrimination switch is marked, it is not always possible to reliably discriminate the corresponding pairwise. Therefore, it is desirable to extend the set cover problem.

Therefore, as shown in Expression (6), the quantification processing unit 106 (selection processing unit 104) totals the quantitative value of the discrimination possibilities by using discrimination redundancy as the pairwise cover number $Z_f^{(k)}$ (calculation of the totalized value as the total value; step S112, quantification step).

$$Z_f^{\left(k \in P_2(C)\right)} = \sum_{i \in f} Y_i^{(k)} \quad \ldots \quad (6)$$

The definition of $Z_f^{(k)}$ is not limited to the definition shown in Expression (6). For example, for the continuous variable version of $-Y_i^{(k)}$, it may be defined as the product of $(1 - Y_i^{(k)})$ as the probability that all discriminations will fail, or the success probability of at least U discriminations may be calculated from $Y_i^{(k)}$ by using a certain appropriate threshold value U. In addition, an average value of individual discrimination possibilities may be calculated. In this way, various totalizing methods can be considered.

Next, from the standpoint that "it is desirable to reduce the bottleneck of the discrimination as much as possible", the optimization processing unit 108 (selection processing unit 104) can return to the feature amount selection problem to the problem of maximizing the minimum pairwise cover number again with the number of the feature amounts to be selected denoted by m, by Expression (7) (step S114: optimization step, optimization processing).

$$\arg\max_{f \subseteq F, |f|=m} \min\left\{Z_f^{(k)} \middle| k \in P_2(C)\right\} \quad \ldots \quad (7)$$

The above is an example of return in a case in which the selected number of the feature amounts is determined (in a case in which the selected number M of the feature amounts is input, that is, in a case in which the selected number input step/processing is performed). On the contrary, the optimization processing unit 108 (selection processing unit 104) may set the threshold value (target threshold value T) for the minimum pairwise cover number (minimum value of the totalized value of the discrimination possibilities) (target threshold value input step/processing), and may select the feature amount so satisfy the threshold value (step S114: optimization step/processing, selection step/processing). In this case, of course, it is desirable that the number of the feature amounts to be selected be smaller, and it is particularly preferable that the number of the feature amounts be the minimum.

Alternatively, various optimization methods, such as combining these two, can be considered.

Since the set cover problem is a field that is actively studied, there are various solutions. The problem of maximizing the minimum cover number, which is an extension of this, can be handled with in almost the same procedure. It should be noted that, since it is generally an NP-complete problem, it is not easy to obtain an exact solution.

Therefore, of course, it is desirable to obtain the exact solution and literally solve the problem of maximizing the minimum pairwise cover number and the problem of achieving the set cover number with the minimum feature amount, but the optimization processing unit 108 (selection processing unit 104) may use a method of increasing the cover number as much as possible, reducing the number of the selected feature amounts as much as possible, or obtaining a local minimum, by a heuristic method.

Specifically, for example, the optimization processing unit 108 (selection processing unit 104) may adopt a simple greedy search procedure. In addition to the minimum pairwise cover number of the currently selected feature set, "a method of sequentially defining i-th smallest i-th rank pairwise cover number, and sequentially selecting the feature amount that maximizes the i-th rank pairwise cover number of smaller i" can be considered.

Further, the importance of the class or the pairwise discrimination may be input (step S112: quantification step, importance input step/processing), and weighting based on the importance may be performed in a case of the optimization (weighting step/processing). For example, Expression (7) can be modified to Expression (8).

$$\arg\max \min\{Zk/wk\} \quad \ldots \quad (8)$$

Here, $w_k$ indicates the importance of the pairwise discrimination. Alternatively, the importance of the class may be designated to obtain $w_k = w_s w_t$ and the like, and the importance of the pairwise may be decided based on the importance of the class. It should be noted that, the calculation expression that reflects the importance of the class of the pairwise based on the product is merely an example, and the specific calculation expression for weighting may be another method to the same effect.

Specifically, for example, in a case in which the discrimination between a disease A and a disease B is particularly important in the discrimination of pathological tissues, while the discrimination between the disease B and a disease C is not important, it is desirable to set a large value to wk = {A, B} and set a small value to wk = {B, C}. As a result, for example, the method of appropriate feature amount selection or class classification (diagnosis) can be provided in a case in which early detection of the disease A is particularly important but a symptom thereof is similar to a symptom of the disease B, and in a case in which early detection of the disease B and the disease C are not important and there is a large difference in the symptoms from each other.

<2.5 Exclusion of Similar Feature Amount>

In general, since the feature amount with high similarity (degree of similarity) that take close values in the entire discrimination target class have high correlation, it is desirable to avoid overlapping selection in consideration of the robustness of the discrimination. In addition, since the optimization search described in the previous term can be made more efficient in a case in which |F| can be reduced, the optimization processing unit 108 (selection processing unit 104) desirably narrows down the feature amount to be considered in advance based on the evaluation result of the similarity (step S110: selection step/processing, similarity evaluation step/processing, priority setting step/processing). Actually, for example, there are hundreds of thousands of methylated sites.

Here, the set of k $I_i = \{k|Y_i^{(k)} = 1\}$ in which $Y_i^{(k)} = 1$ for the feature amount i is called the "switch set". From this switch set, it is possible to consider the similarity (or degree of similarity) of the feature amounts, that is, the equivalence relationship (overlap relationship) and the inclusion relationship of the feature amounts.

For the feature amount i, all l in which $I_i = I_l$ is collected, and the equivalent feature set $U_i$ is created as shown in Expression (9). In addition, all l as $I_i \supset I_l$ is collected to create an inclusion feature set $H_i$ as shown in Expression (10).

$$U_i = \left\{ i, l_1^{(i)}, l_2^{(i)}, \dots \middle| I_i = I_{l_*^{(i)}} \right\} \quad \dots \quad (9)$$

$$H_i = \left\{ l_1^{(i)}, l_2^{(i)}, \dots \middle| I_i \supset I_l \right\} \quad \dots \quad (10)$$

Since the equivalent feature set is obtained by grouping the overlapping feature amounts and the inclusion feature set is obtained by grouping the dependent feature amounts, the feature amount having high similarity can be excluded by narrowing down the feature amounts to one representative feature amount. Therefore, for example, the whole feature set F may be replaced with the similar exclusion feature set as in Expression (11).

$$F' = F \setminus \left\{ \left\{ l^{(i)} \middle| \exists U_i, l^{(i)} \neq i, l^{(i)} \in U_i \right\} \cup \left\{ l^{(i)} \middle| \exists H_i, l^{(i)} \in H_i \right\} \right\} \quad \dots \quad (11)$$

Of course, the selection processing unit 104 may consider only one of the equivalent feature set or the inclusion feature set as the similarity, or may create another index. For example, a method of obtaining a vector distance between the feature amounts (distance between discrimination possibility vectors) and regarding the vector distance equal to or less than a certain threshold value as the similar feature amount can be considered. In addition to the simple distance, any distance or a metric value equivalent thereto may be introduced, such as normalizing the discrimination possibilities of a plurality of feature amounts and then calculating the distance.

Further, although the narrowing down is performed in the above, the selection processing unit 104 may use a method of lowering the selection priority (priority) of the feature amount for which the similar feature amount is already selected in a case of the optimization search (priority setting step) to decide the ease of selection. Of course, a method of raising the selection priority (priority) of the feature amount having a low degree of similarity to the already selected feature amount (priority setting step) may be used.

<2.6 Introduction of Discrimination unneeded Pairwise (class set)>

The class binary relationship extends to $|P_2(C)| = {}_NC_2$ for the given class number N. This is simply the binary relationship of the class, but there may be pairwise that does not need to be discriminated in practice.

For example, in a case of assuming a cancer diagnosis problem (see examples described below), the discrimination between the cancer tissues and the discrimination between the cancer tissue and the normal tissue is essential, but the discrimination between the normal tissues is not needed.

Therefore, the selection processing unit 104 may partially suppress the pairwise expansion of the class binary relationship. That is, the given class C = {c|c ∈ $C_T$, $C_N$} is divided by the class set $C_T$ that needs to be discriminated and the class set $C_N$ that does not need to be discriminated (first discrimination unneeded class group) to make consideration between $C_T$ and $C_T$, and between $C_T$ and $C_N$ (pairwise expansion), while the pair of $C_N$ is excluded from the class binary relationship (step S110: selection step, first marking step/processing, first exclusion step/processing). That is, the selection processing unit 104 calculates $P_2(C)'$ by Expression (12), and replaces the existing $P_2(C)$ with $P_2(C)'$.

$$P_2\left(C\right)^{\cdot} = P_2\left(C\right) \backslash \left\{\left\{s,t\right\} \big| s \neq t \in C_N\right\} \quad \dots (1\ 2)$$

It should be noted that, two or more such divisions or marks may be present.

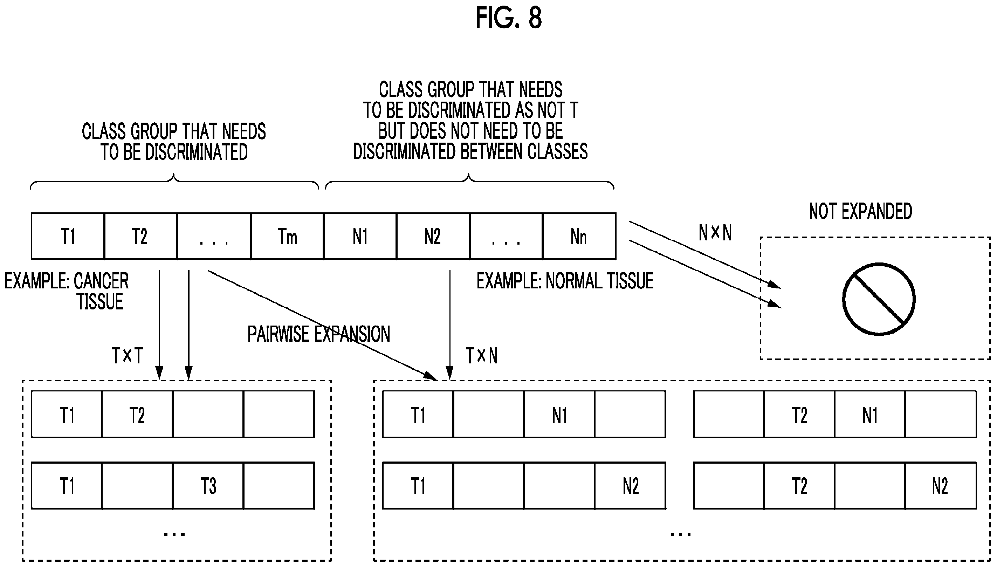

Fig. 8 is a diagram showing an aspect in which the pairwise expansion is partially suppressed. In the example of Fig. 8, classes T1, T2, ..., Tm are a class group (for example, cancer tissues) that needs to be discriminated between the classes, and classes N1, N2, ..., Nn are a class group (for example, normal tissues) that needs to be discriminated as "not T (not the cancer tissue)" but does not need to be discriminated between the classes.

In this case, the selection processing unit 104 performs the pairwise expansion between the classes T (for example, classes T1 and T2 and classes T1 and T3) and between the class T and the class N (for example, classes T1 and N1 and classes T1 and N2), but does not perform the pairwise expansion between the classes N.

<2.7 Introduction of Subclass from Sample Clustering>

Even in a case in which the correct answer class label is given to the sample, there is a case in which a plurality of groups with different appearances are actually mixed in the sample of the same class nominally. Even in a case in which it is sufficient to discriminate the nominal class, the feature amounts do not always follow the same distribution parameter, so that the discrimination switch cannot be correctly given.

For example, there are subtypes of the cancer, and some cancers of the same organ have different appearances [Holm, Karolina, et al., 2010]. It should be noted that, in a case in which application to the screening test (combined with detailed tests) is assumed, it is not needed to discriminate the subtype.

Therefore, in order to correspond to the subtype, a special class unit called the subclasses, which do not need to be discriminated from each other, may be introduced (step S110: selection step, subclass setting step/processing, second marking step/processing).

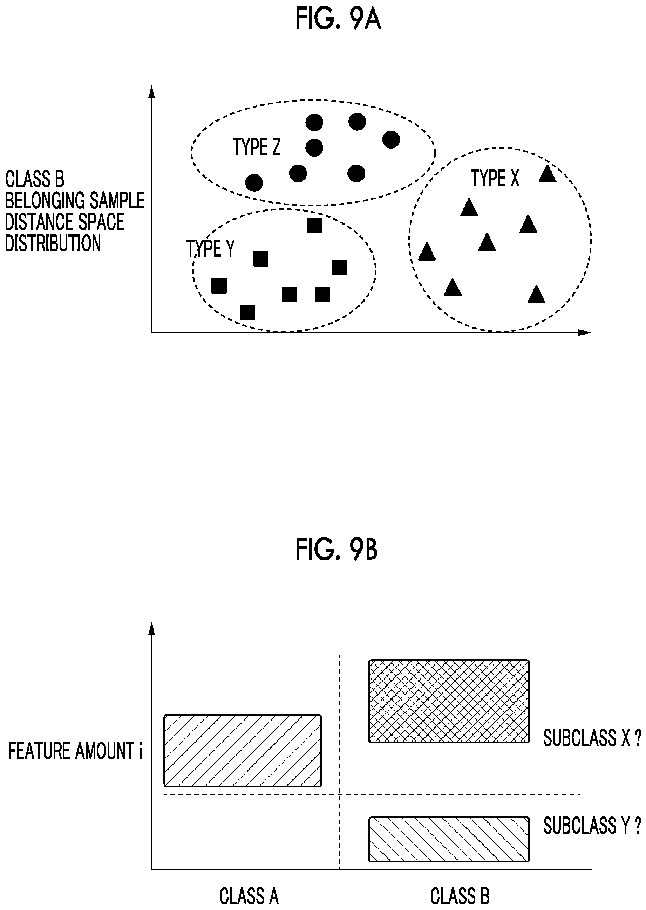

The subclass can be automatically configured from samples. It should be noted that, since it is difficult to perform identification from a single feature amount, a method can be considered in which the selection processing unit 104 clusters (forms a cluster) the sample by the total feature amount (given feature amount) for each class and divides an appropriate cluster number L (or minimum cluster size $n_C$) to make the subclass correspond to the cluster. For example, as shown in Fig. 9A, the samples belonging to a certain class (here, class B) are clustered using all the feature amounts, and based on the result, the samples are divided into the subclasses X and Y as shown in Fig. 9B. In this example, in a case in which the class B is divided into the subclasses X and Y, the feature amount i can be used for the discrimination between the class A and the subclass Y of the class B. It should be noted that, there is a case in which a certain class is accidentally divided into a plurality of subclasses, and in that case, it is nonsense to forcibly consider it as the "subclass".

It should be noted that there are various clustering methods, clustering may be performed by another method, and clustering criteria may be set in various ways.

For example, in a case in which the class J is divided into {$J_1$, $J_2$, ... , $J_L$} (second discrimination unneeded class group), the given class C = {1,2, ... , J, ... , N} can be extended by Expression (13).

$$C^{+J} = \left\{1, 2, ..., J_1, J_2, ..., J_L, ..., N\right\} \quad \dots (1\ 3)$$

As in the previous term, the class binary relationship is replaced by Expression (14) by excluding the pair of subclasses that do not need to be discriminated (second exclusion step).

$$P_2\left(C^{+J}\right)^{'-J} = P_2\left(C^{+J}\right)^{'} \backslash \left\{\left\{s,t\right\} \big| s \neq t \in J_*\right\} \quad \dots (1\ 4)$$

It should be noted that the final class binary relationship applied sequentially including the previous term $C_N$ is referred to as $P_2(C^{+C})'^{-C}$.

<2.8 Summary of Procedure of Feature Selection Method>

The procedure of the feature selection method (selection step by the selection processing unit 104, selection processing) proposed by the inventors of the present application is summarized.

(i) In the given class set C, the class set $C_N$ that does not need to be discriminated is set.
(ii) The samples are clustered for each class with all the feature amounts to make each obtained cluster correspond to the subclass (subclass is special classes that do not need to be discriminated from each other).
(iii) The pairwise expansion $P_2 (C^{+C})'^{-C}$ of all the class binary relationships, which are discrimination targets, excluding those that do not need to be discriminated is determined.
(iv) The distribution parameter from the sample belonging to each class is estimated and the significant difference in the feature amount in the class pair k = {s, t} is determined by the statistical test to assign 0/1 to the discrimination switch $Y_i^{(k = \{s, t\})}$.
(v) From the discrimination switch, the equivalent feature amount set and the inclusion feature amount set are configured to create the similar exclusion feature set F'.
(vi) The feature set f (feature amount set) that maximizes the minimum value of the pairwise cover number $Z_f^{(k)}$ obtained from the sum of the discrimination switches is selected from the F' for the entire pairwise expansion $P_2(C^{+C})'^{-C}$ of the discrimination target class.

It should be noted that, the above i to vi are comprehensive examples, and it is not always needed to implement all of the above i to vi, and there may be a procedure of partially rejecting the above i to vi. In addition, of course, the configuration may be used in which the alternative method specified or suggested in each section is used. It should be noted that the multi-class classification device 10 may execute only the steps of the feature amount selection method (feature amount selection method, feature amount selection processing) to obtain the feature amount set used for the multi-class classification.

<3. Multi-Class Classification Method>

In the present chapter, the processing (step S120: determination step, determination processing) performed by the class determination processing unit 114 (determination processing unit 110) will be described. First, a configuration example (class determination step, determination step) of the binary-class classifier (binary-class discriminator) based on the selected feature amount (selected feature amount group, feature amount set) will be described. Next, an example (class determination step, determination step) of a method of configuring (configuring the multi-class discriminator that uses the selected feature amount group in association with the pairwise coupling) the multi-class classifier (multi-class discriminator) from the binary-class classifier, by two-stage procedure of (1) brute force match ranking and (2) final tournament match will be described.

<3.1 Configuration of Binary-Class Classifier>

The fact that the feature amount that contributes to the pairwise discrimination is selected will be utilized. Therefore, the binary-class classifier can be configured only from the combination of the pairwise and the feature amount marked with the discrimination switch (each of the binary-class discriminator that use the selected feature amount group in association with each pairwise coupling is configured). It should be noted that in a case of the class classification, the acquisition processing unit 112 acquires the value of the feature amount of the selected feature amount group (step S122: acquisition step, acquisition processing).

For example, the class determination processing unit 114 (determination processing unit 110) can decide the discrimination switch state $y_i^{(k = (s, t), j)}$ for the class pairwise {s, t} of the given sample j (belonging class is unknown) and the selected feature amount i by comparing with the learning distribution (step S124: class determination step, see Figs. 7A and 7B). First, the distribution is estimated from the learning data to determine the significant difference (whether it is the state shown in Fig. 7A or the state shown in Fig. 7B), and in a case of "there is the significant difference", a threshold value is set in advance. Then, the class determination processing unit 114 estimates the belonging distribution (or whether does not belong to) from the value of the feature amount in a case in which the given sample is classified only in a case in which "there is the significant difference" is selected, to decide the discrimination switch state value as shown in Expression (15) (step S124: class determination step).

$$y_i^{(k=(s,t),j)} = \begin{cases} +1 \text{ if } Y_i^{(k)} = 1 \text{ and } x_i^{(?,j)} \sim D\left(\theta_i^{(s)}\right) \\ 0 \text{ if } Y_i^{(k)} = 0 \text{ or } x_i^{(?,j)} \nsim D\left(\theta_i^{(s)}\right), D\left(\theta_i^{(t)}\right) \quad \dots \quad (15) \\ -1 \text{ if } Y_i^{(k)} = 1 \text{ and } x_i^{(?,j)} \sim D\left(\theta_i^{(t)}\right) \end{cases}$$

It should be noted that the "?" in Expression (15) indicates that the class to which the sample x belongs is unknown. In addition, in a case in which the value of the feature amount of the sample is NA, y is set to 0.

The class determination processing unit 114 (determination processing unit 110) totals the above values to calculate a discrimination score $r_j(s, t)$, and configures the binary-class classifier $B_j(s, t)$ as shown in Expressions (16) and (17) (step S124: class determination step)

$$r_j(s,t) = \sum_{i \in f} y_i^{(k=\{s,t\},j)} \quad \dots \quad (16)$$

$$B_j(s,t) = \begin{cases} s \text{ if } r_j(s,t) > 0 \\ t \text{ if } r_j(s,t) < 0 \end{cases} \quad \dots \quad (17)$$

<3.2 Procedure (1) of Multi-Class Classification: Brute Force Match Ranking>

The class determination processing unit 114 (determination processing unit 110) can further total the above-described discrimination scores (note that, in order to normalize the number of discrimination switches, it is desirable to take a code value) to calculate a class score (pair score) as shown in Expression (18) (step S124: class determination step).

$$R_j(s) = \sum_{\left\{ t \middle| \{s,t\} \in P_2\left(C^{+C}\right)^{i-C} \right\}} \text{sgn}\left(r_j(s,t)\right) \quad \dots \quad (18)$$

This class score indicates "how similar the unknown sample j is to the class s". Further, the class determination processing unit 114 (determination processing unit 110) lists the discrimination candidate classes in descending order of the class score and creates a brute force match ranking G (step S124: class determination step). In a case of the creation, replacement processing (replace with +1 in a case in which the class score is positive, leave the value at ± 0 in a case in which the class score is zero, and replace the value with -1 in a case in which the class score is negative) may be performed.

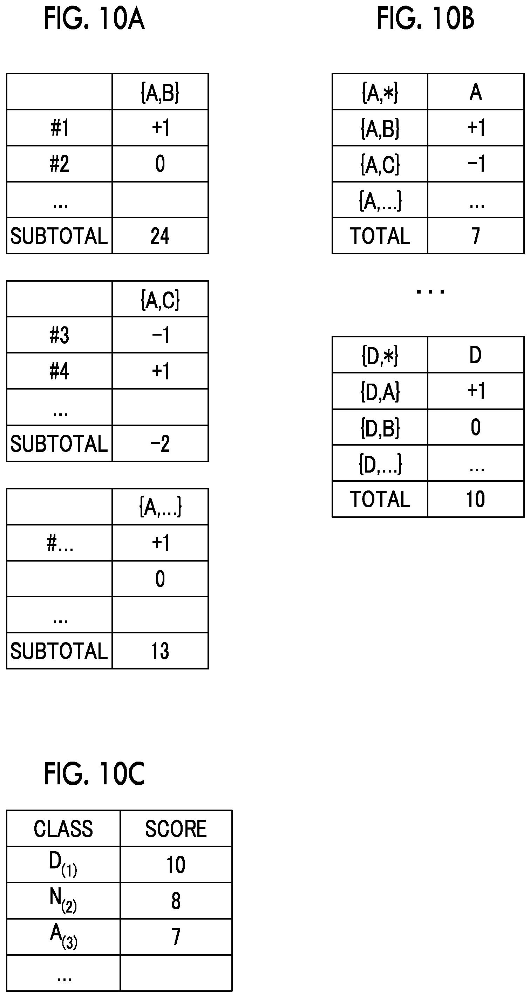

Figs. 10A to 10C are diagrams showing aspects of creating the brute force match ranking. First, as shown in Fig. 10A, the class determination processing unit 114 totals the code value (Sgn ($r_j$(s, t)) in Expression (17)) of the discrimination score for each class pair ({ A, B}, {A, C}, ...). For example, "the sample is similar to the class A in terms of the value of feature amount #1 (code value = +1), and cannot be said to be any of the class A or B in terms of the value of feature amount #2 (code value = 0) ... " for the class pair {A, B}, and the subtotal is 24. Therefore, it can be said that "the sample is similar to A in the classes A and B" (as the subtotal value is positive and the absolute value is larger, the degree of similarity is higher). In addition, "the sample is similar to the class C in terms of the value of feature amount #3 (code value = - 1), and is similar to the class A in terms of the value of feature amount #4 (code value = +1) ... " for the class pair {A, C}, and the subtotal is -2. Therefore, it can be said that "the sample is not similar to any of the class A or C (or is slightly similar to the class C)".

In a case in which the subtotal is calculated for all the class pairs in this way, the result shown in Fig. 10B is obtained. For example, {A, *} is the "comparison result between the class A and all other classes", and the total score after replacement described above is 7. Similarly, the total for the class D is 10. Then, the class determination processing unit 114 lists (ranks) the discrimination candidate classes from this total as shown in Fig. 10C. In this example, the totals for the classes D, N, and A are 10 ,8, 7, respectively, the class D is first rank, the class N is second rank, and the class A is third rank.

<3.3 Procedure (2) of Multi-Class Classification: Final Tournament Match>

In multi-class classification including the present problem, the discrimination between the similar classes often becomes a performance bottleneck. Therefore, in the present invention, the feature amount group (feature amount set) capable of discriminating all the pairwise including the similar classes is selected.

On the other hand, in the brute force match ranking G, although it is expected that highly similar classes will gather near the top rank, most of the class scores are determined by comparison with the lower rank classes. That is, the ranking near the top rank (ranking between the classes D, N, and A in the examples of Figs. 10A to 10C) is not always reliable.

Therefore, the class determination processing unit 114 (determination processing unit 110) can decide the final discrimination class based on an irregular tournament match $T_j$ of g higher rank classes in the brute force match ranking, as shown in Expression (19) (step S124: class determination step).

$$T_j\left(G_1, G_2, ..., G_g\right) = T_j\left(G_1, ..., G_{g-2}, B_j\left(G_{g-1}, G_g\right)\right)$$

$$= ... = B_j\left(G_1, B_j\left(G_2, ..., B_j(G_{g-1}, G_g)...\right)\right) \qquad ... \ (1\,9)$$

That is, the class determination processing unit 114 applies the binary-class classifier again to the pairwise of the lower rank two classes from the g classes at the higher rank of the list, determines survival to reduce the number of lists by one, and sequentially performs the same procedures (finally, the G top rank class is compared with the surviving class).

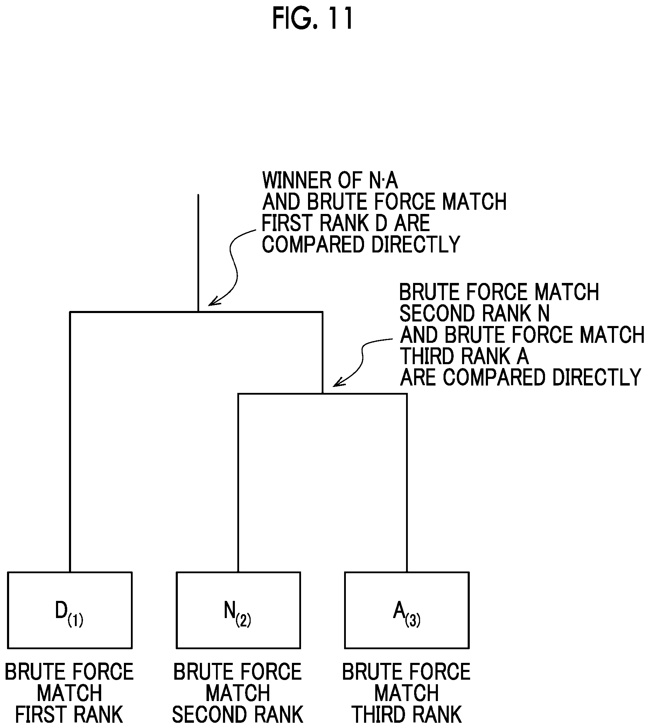

For example, as shown in Fig. 11, the class score is calculated for the lower rank two classes N and A from the three classes (classes D, N, and A) at the higher rank of the list to determine survival (class N or A), the class score is calculated in the same way for the class D, which is the top rank class in the brute force ranking, and the surviving class. It should be noted that, "how many ranks in the brute force ranking are targeted for the final tournament match (up to third rank in the example of Fig. 11)" is not particularly limited.

<3.4 Configuration of Other Multi-Class Classifiers>

It should be noted that the above description is an example of the classifier configuration, and various machine learning methods may be used in addition to the above example. For example, the configuration may be basically a random forest configuration, in which only those for which the discrimination switch of the selected feature amount is effective are used (determination step) in the decision tree in the middle. Specifically, the class determination processing unit 114 (determination processing unit 110) may configure the decision tree that uses the selected feature amount group in association with each pairwise coupling, and may combine one or more decision trees to configure the multi-class discriminator (step S124: class determination step). In this case, the class determination processing unit 114 may configure the multi-class discriminator as the random forest by combining the decision tree and the decision tree (step S124: class determination step).

<4. Output>

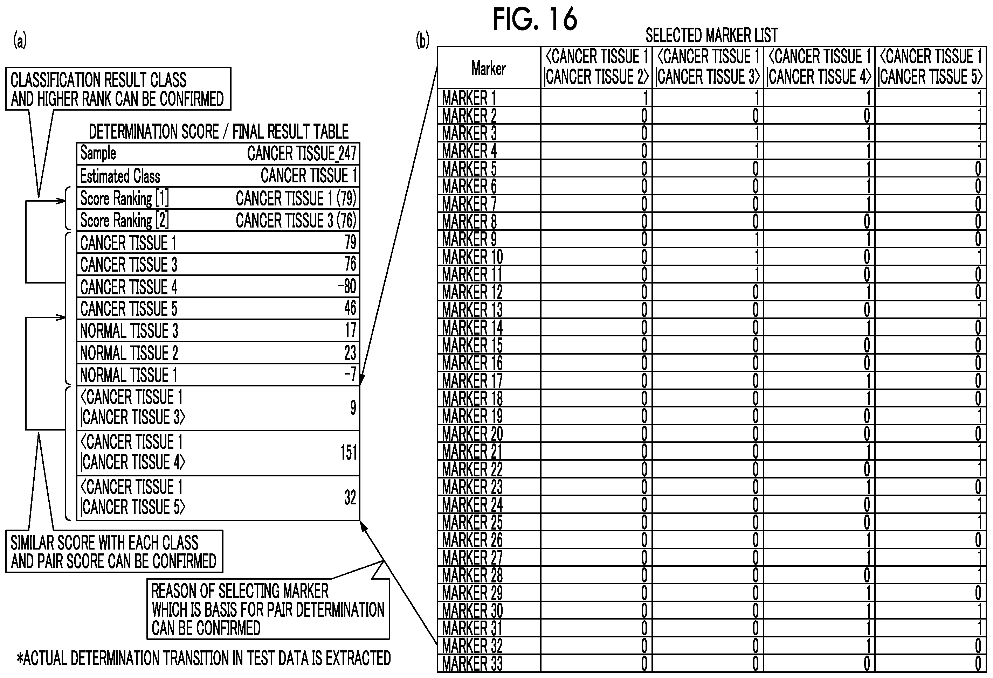

The output processing unit 115 can output the input data, the processing condition described above, the result, and the like in accordance with the operation of the user via the operation unit 400 or without the operation of the user. For example, the output processing unit 115 can output the input learning data set, the selected feature amount set, the result of the brute force match ranking or the final tournament match, and the like by the display on the display device, such as the monitor 310, the storage in the storage device, such as the storage unit 200, the print by a printer (not shown), and the like (output step, output processing; Fig. 16 will be described below).

<5. Test Data and Examples>

The inventors of the present application select 8 types (large intestine cancer, stomach cancer, lung cancer, breast cancer, prostate cancer, pancreatic cancer, liver cancer, and cervical cancer) of the cancer, which are diagnosis targets. Since these cancers account for about 70% of Japanese cancers [Hori M, Matsuda T, et al., 2015], these cancers are considered to be an appropriate target for the early screening test.

In addition, since normal tissue needs to cover everything that can flow out into the blood, a total of 24 possible types, such as blood, kidney, and thyroid gland, are listed in addition to the organs corresponding to the 8 types of the cancer.

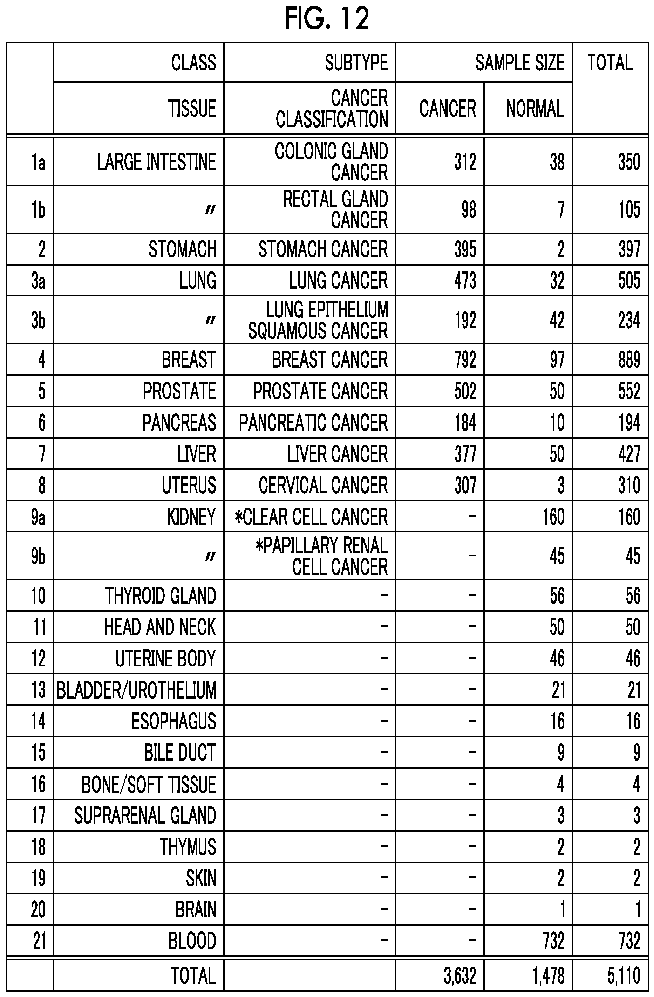

A total of 5,110 open data samples including the measurement values of the methylated site are collected assuming the discrimination of the extracted cell aggregates (living body tissue piece) by positioning as a feasibility study (Fig. 12).

For the cancer tumor and the normal organ (excluding blood), 4,378 samples are collected from the registered data of "The Cancer Genome Atlas" (TCGA) [Tomczak, Katarzyna, et al., 2015]. In addition, 732 samples of the blood are also collected [Johansson, Asa, Stefan Enroth, and Ulf Gyllensten, 2013].

All sample belonging classes (origin tissues including cancer and non-cancer distinction) are assigned in accordance with the registered annotation information.

In addition, the total number of methylated measurement values is 485,512 sites, but is 291,847 sites, excluding those for which all sample values cannot be measured (NA). It should be noted that, in the registered data described above, the data subjected to the post-processing, such as normalization, is used as it is.

Further, the entire data set is mechanically divided into equal parts, one data set is used as the learning data set and the other data set is used as a test data set.

The trial tasks set in the present example are as follows.

i. About 5,000 samples of the data sets are prepared

Allocation class (32 in total): Cancer (8 types) or normal tissue (24 types)
Feature amount (methylated site): about 300,000 items

ii. From the above half of the learning data set, at maximum 10 to 300 items of methylated site (omics information, omics switch-like information) that can be used for the discrimination are selected in advance (and learning with parameter, such as subclass division or distribution parameter)
iii. Answer is made (one sample at a time) to the discrimination problem of the given sample (particularly from the other half of the test data set)

Input: selected methylated site measurement value of the sample (at maximum 300 items corresponding to the selection in ii)
Output: 9 types of estimated class = "cancer + origin tissue (select from 8 types)" or "non-cancer (only 1 type)" are selected

It should be noted that, in the example, the following method is adopted as a related-art method to be compared with a proposal method (method according to the embodiment of the present invention).

·Feature selection method: shannon entropy criteria with methylated site research cases [Kadota, Koji, et al., 2006; Zhang, Yan, et al., 2011]
·Multi-class classification: naive bayes classifier (simple but known for its high performance [Zhang, Harry, 2004])

<5.1 Comparison Result between proposal method and Related-Art Method>

<5.1.1 Discrimination Accuracy of Test Data>

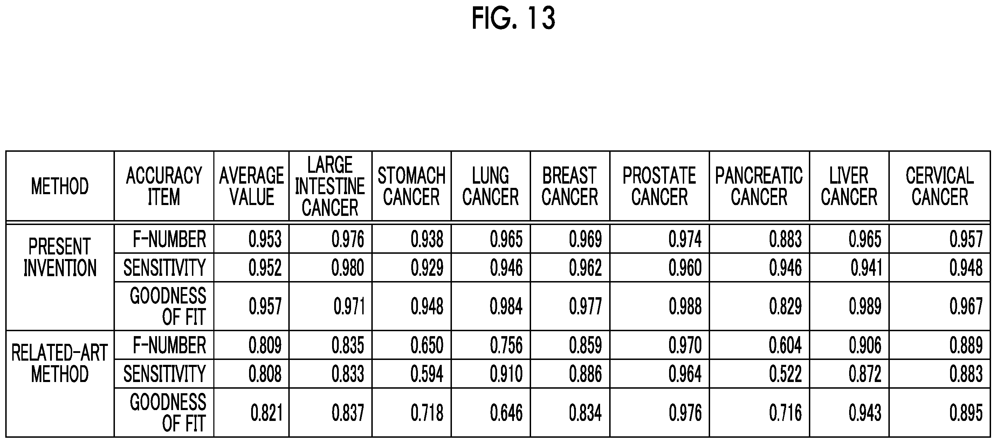

Learning with the learning data is performed, 277 sites (omics information, omics switch-like information) are selected, the discrimination accuracy of the test data is confirmed, and the proposal method (multi-class classification method according to the embodiment of the present invention) is compared with the related-art method (Fig. 13). As a result, it is shown that the proposal method has high discrimination accuracy in all items.

The average F-number of the related-art method is 0.809, while the average F-number of the proposal method reaches 0.953. In addition, in the related-art method, some lung cancer, pancreatic cancer, stomach cancer, and the like have an F-number/sensitivity/goodness of fit of less than 0.8, but the proposal method achieves 0.8 or more in all items.

<5.1.2 Robustness of Discrimination>

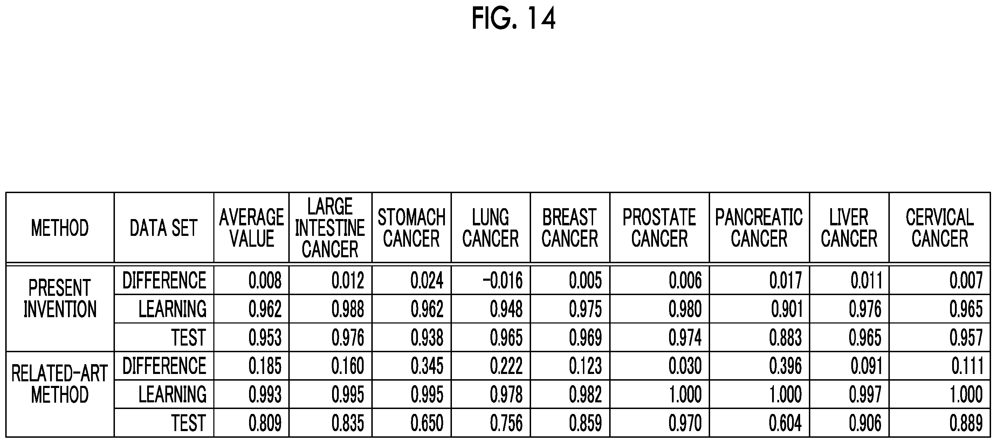

The robustness of the discrimination is confirmed by the average F-number difference between the learning and the test in the previous term, and the proposal method is compared with the related-art method (Fig. 14). As a result, it is shown that the robustness of the proposal method is excellent (reduction of the F-number is 0.008).

It can be seen that the related-art method shows an almost perfect average F-number of 0.993 for the learning data, and the accuracy of the test data is greatly reduced (difference 0.185), resulting in over-learning.

On the other hand, in the proposal method, the reduction in the average F-number is only 0.008. In addition, the discrimination ability of the pancreatic cancer has a relatively low value (F-number 0.883) within the proposal method, but also has a relatively low value (0.901) during learning. This proposal method suggests that the discrimination accuracy and tendency in the test data can be predicted to some extent at a stage of learning completion.

<5.1.3 Relationship between Number of Selected Features and Discrimination Accuracy>

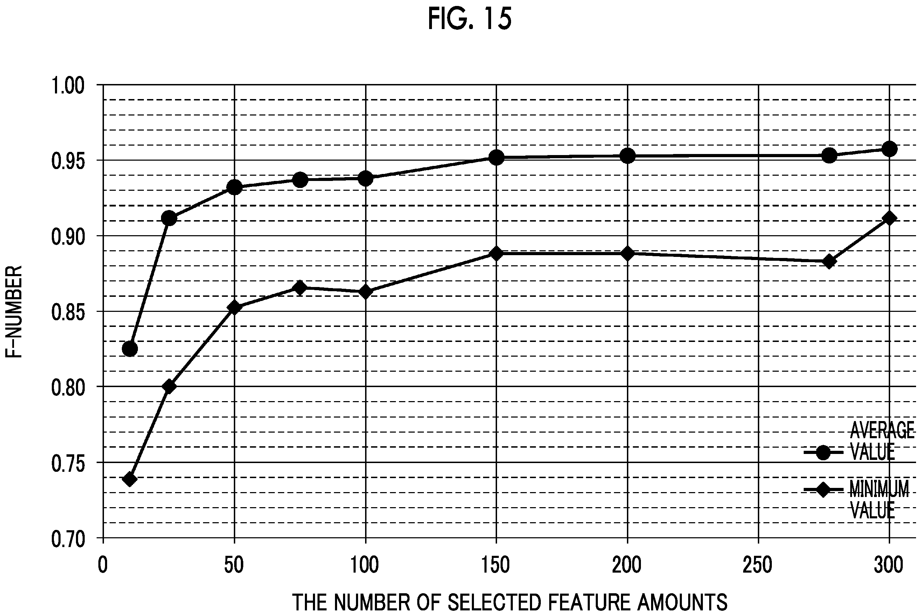

The relationship between the number of the selected feature amounts and the discrimination accuracy (F-number) is confirmed (Fig. 15). As a result, it can be seen that the discrimination accuracy is remarkably improved in a case in which 50 to 100 pieces are selected, and tends to be saturated in a case in which 150 to 300 pieces are selected.
Therefore, it is shown that, in the cancer diagnosis problem to discriminate "whether it is cancer or non-cancer" and the origin tissue particularly from the methylated pattern of cfDNA, the discrimination ability is not sufficient with 10 feature amount selections, the multi-measurements of at least 25 to 100 items are required (therefore, in the multi-class classification problem with a large number of classes, the number of the feature amounts (selected feature amount groups) selected in the selection step (selection processing) is preferably 25 or more, more preferably 50 or more, and still more preferably 100 or more).

<5.1.4 Exclusion of Similar Feature Amount and Introduction of Discrimination unneeded Pairwise>

In the proposal method, the similar feature amounts are not selected (similarity evaluation step, similarity evaluation processing). In addition, discrimination unneeded pairwise is introduced.
There are a total of 291,847 effective methylated sites (feature amounts in the present problem), but among 291,847 effective methylated sites, 59,052 similar feature amounts (equivalence relationship, inclusion relationship) can be specified and reduced by excluding from the target (reduction by 20.2%). In addition, since the original 32 classes are divided into 89 classes by sample clustering, the total number of simple pairwise is 4,005. Of these, the non-target pairwise between 551 normal tissues and cancer subclasses can be reduced (reduction by 13.8%).
At the same time, the search space can be reduced by 31.2%. It can be confirmed that the efficiency of the discrimination switch combination search is improved by excluding the similar feature amounts and introducing the discrimination unneeded pairwise.

<5.1.5 Subclass Division>

In the proposal method, sample clustering is introduced to internally divide the given class into the subclasses. Since the combination with the discrimination unneeded pairwise is also important, the effect of both is confirmed.
For comparison, a trial is performed in which subclass division is not performed, discrimination unneeded pairwise of the feature selection is not introduced, and other procedures are the same. As a result, even in a case of being limited to the cancer tissue, the correct answer rate of the discrimination is reduced from the original 95.9% to 85.6% (since there are 24 types of the normal tissues without division, particularly to confirm the effect of the subclass division, the comparison is limited to the cancer tissue).
It can be confirmed that highly accurate discrimination is realized by introducing subclass division and discrimination unneeded pairwise.

<5.1.6 Combined Use of Final Tournament Match>

In the proposal method, in the multi-class classification, the brute force match ranking (in the present section, the first rank class is called the "qualifying top class") and the final tournament match are used in combination.
In the 2,555 test data, there are 278 cases in which the qualifying top class does not match the correct answer class. Among these, there are 162 cases that can be corrected to the correct discrimination by the final tournament match. On the other hand, there are 19 opposite cases (the qualifying top class matches the correct answer class, but it is changed to the wrong discrimination by the final tournament match).
That is, by using the final tournament match in combination, it is possible to correct 51.4% by subtracting the discrimination error of the qualifying top class, and improve the overall correct answer rate by 5.6%. The configuration can be confirmed in which the performance of the binary-class classifier is skillfully brought out by pairwise discrimination.
In the proposal method, the discrimination procedure, the comparative study class, and the dependent feature amount are clear. Therefore, it is possible to trace back the discrimination result and easily confirm and describe the difference with the feature amount or the threshold value which is the basis. It can be said that it is an "AI that can be described" that is particularly advantageous for application to medical diagnosis that requires a basis for the discrimination.
Fig. 16 is a table showing a shown example of a basis for the discrimination (extracted from the actual determination transition in the test data). The higher rank class and the result of the classification result and the score are shown in a portion (a) of Fig. 16. In the example of Fig. 16, it can be seen that the sample is classified into "cancer tissue 1" and its score is 79, and the next similar sample is "cancer tissue 3" and its score is 76.
In the same manner, each class score $R_i(s)$ can be confirmed in the 7 rows from the row of "cancer tissue 1" to the row of "normal tissue 1". In addition, each class pairwise discrimination score $r_j(s, t)$ can be confirmed in the three rows from the row "<cancer tissue 1|cancer tissue 3>" to the row "<cancer tissue 1|cancer tissue 5>".

In addition, in the table shown a portion (b) of Fig. 16, a list of "how the selected feature amount (described as the marker in the table) list contributes to each discrimination score" can be confirmed. Of course, in addition to the distribution map of the learning data as shown in Fig. 7A, visualization such as plotting the values of each sample on the figure may be added.

As described above, with the proposal method (the present invention), after the classification (selection), the processing steps are traced in the reverse order, and each score or the like is shown, so that the basis for the discrimination can be confirmed and visualized. As a result, the reliability degree of the final discrimination result can be estimated from the similar class score, the discrimination score, or the like of other candidates. In addition, specifying the feature amount that is the basis can be connected to the consideration after classification by its interpretation.

<Relationship between Number of Selected Feature Amounts and Minimum Cover Number>

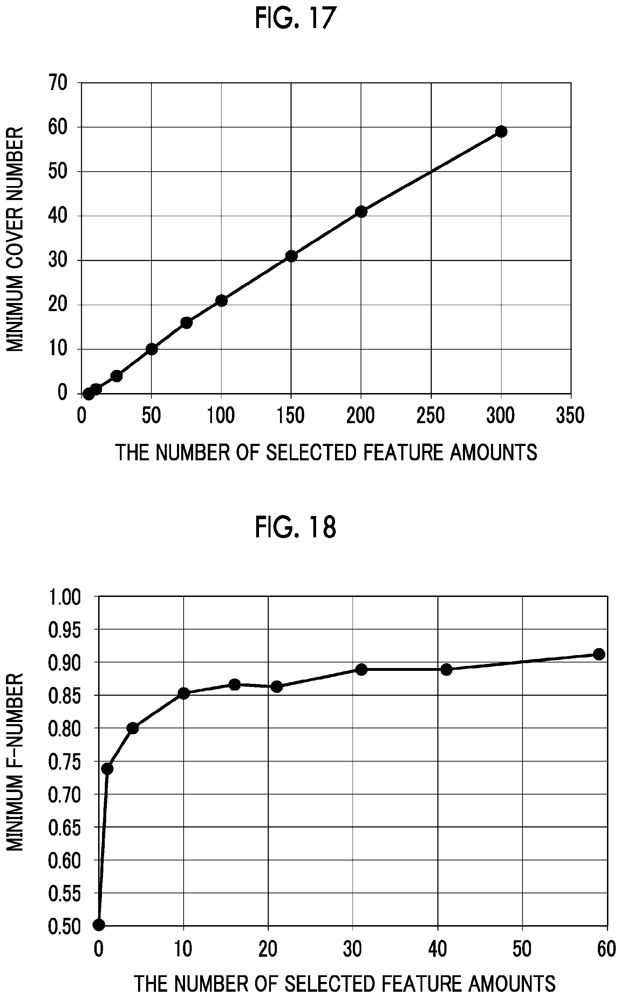

The relationship between the number of the selected feature amounts and the minimum cover number in the example described above is shown in a graph of Fig. 17.

$$\left(\min\left\{Z_f^{(k)}\right\}\right) \quad \cdots \quad (20)$$

Here, a linear relationship with a slope of generally 1/5 is obtained, and it means that, for advanced multi-class classification problems such as cancer 8 classes/normal 24 classes, and with internal subclass division, the feature amount set that covers all the class discriminations can be selected for generally every five selections. That is, the feature selection of the method according to the embodiment of the present invention is returned to the set cover problem, and it is shown that the effect of extension is great and the minimum cover number can be efficiently improved in the multi-class classification problem. In addition, it can be seen that, from Fig. 17, by finely adjusting the obtained feature amount set, the feature amount set that shows high discrimination ability with a small part of the total feature amounts, specifically, 5 times or less of the needed minimum cover number can be created, and the feature amount set that satisfies the minimum cover number with such a small number is greatly valuable.

<Relationship between Minimum Cover Number and Minimum F-Number>

The relationship between the minimum cover number in the selected feature amount set and the minimum F-number (the minimum value of the discrimination ability F-number in the test data in the discrimination target class) is shown in a graph of Fig. 18.

$$\left(\min\left\{Z_f^{(k)}\right\}\right) \quad \cdots \quad (21)$$

From this, it can be read that, in a case in which the minimum cover number is 0, almost no performance can be obtained, the minimum F-number becomes 0.8 at around 5, the minimum F-number becomes 0.85 at around 10, and 0.9 at around 60. That is, first, it can be seen that almost no performance can be obtained unless the feature amount set having the minimum cover number of at least 1 or more is selected. In addition, the detailed criteria for the F-number actually needed vary depending on the problem, but 0.80, 0.85, and 0.90 are easy-to-understand criteria, so it can be seen that the feature amount set having the minimum cover number of 5, 10, or 60 or more is valuable. In addition to the previous term (relationship between the number of the selected feature amounts and the minimum cover number), "achieving the cover number by a relatively small number of the selected feature amounts (5 times or less of the presented minimum cover number)", which can be realized by the present invention, is particularly valuable.

It should be noted that, the example about "methylated site and living body tissue classification" described above is merely one of specific examples. The method according to the embodiment of the present invention is sufficiently generalized and can be applied to any feature amount selection and multi-class classification outside the field of biotechnology. For example, in a case of performing the class classification of people in the image (for example, Asia, Oceania, North America, South America, Eastern Europe, Western Europe, Middle East, and Africa), the feature amount can be selected by the method according to the embodiment of the present invention from a large number of the feature amounts, such as the size and shape of the face, skin color, hair color, and/or the position, the size, and the shape of the eyes, nose, and mouth, and the multi-class classification can be performed by using the selected feature amount. In addition, the method according to the embodiment of the present invention may be applied to the feature amount selection and the class classification of agricultural, forestry and fishery products, industrial products, or various statistical data.

The embodiments and other examples of the present invention have been described above, but the present invention is not limited to the aspects described above and can have various modifications without departing from the scope of the present invention as defined by the claims

Explanation of References

    10: multi-class classification device
    100: processing unit
    102: input processing unit
    104: selection processing unit
    106: quantification processing unit
    108: optimization processing unit
    110: determination processing unit
    112: acquisition processing unit
    114: class determination processing unit
    115: output processing unit
    116: CPU
    118: ROM
    120: RAM
    200: storage unit
    300: display unit
    310: monitor
    400: operation unit
    410: keyboard
    420: mouse
    NW: network
    S100 to S124: each processing of multi-class classification method

**Claims**

1.  A computer-implemented feature amount selection method of selecting a feature amount group to be used for determining which of N (two or more) classes a sample belongs to, the method comprising:

    an input step (S100) of inputting a learning data set including a known sample group belonging to a given class, which is a target, and a feature amount group of the known sample group; and
    a selection step (S110) of selecting a feature amount group needed for class determination for an unknown sample of which a belonging class is unknown, from the feature amount group based on the learning data set, wherein the selection step (S110) includes

        a quantification step (S112) of quantifying, by a pairwise coupling that combines two classes among the N classes, a discrimination possibility between the two classes in accordance with each feature amount of the selected feature amount group by using the learning data set, and
        an optimization step (S114) of totalizing the quantified discrimination possibilities for all the pairwise couplings and selecting a combination of the feature amount groups for which a result of the totalization is optimized, **characterized by**
        a first marking step of marking a part of the given classes as first discrimination unneeded class groups that do not need to be discriminated from each other, and
        a first exclusion step of excluding the pairwise coupling in the marked first discrimination unneeded class groups from pairwise couplings to be expanded.

2.  The feature amount selection method according to claim 1,
    wherein the selection step further includes

a similarity evaluation step of evaluating similarity between the feature amounts based on the discrimination possibility for each pairwise coupling of each feature amount, and
a priority setting step of setting a priority of the feature amount to be selected, based on an evaluation result of the similarity.

3. The feature amount selection method according to claim 2,
wherein the similarity is an overlap relationship and/or an inclusion relationship of the discrimination possibility for each pairwise coupling.

4. The feature amount selection method according to claim 2 or 3,
wherein the similarity is a distance between discrimination possibility vectors for each pairwise coupling or a metric value in accordance with the distance.

5. The feature amount selection method according to any one of claims 1 to 4, further comprising:

a selected number input step of inputting a selected number M of the feature amounts in the selection step,
wherein the optimization is maximizing a minimum value of a totalized value in all the pairwise couplings in accordance with M selected feature amounts.

6. The feature amount selection method according to any one of claims 1 to 5, wherein the optimization step includes

an importance input step of inputting importance of the class or pairwise discrimination, and
a weighting step of performing weighting based on the importance in a case of the totalization.

7. A non-transitory computer-readable recording medium comprising instructions which, when executed by a computer, cause the computer to carry out the steps of the method according to any of claims 1 to 6.

8. A multi-class classification method of determining, in a case in which N is an integer of 2 or more, which of N classes a sample belongs to, from a feature amount of the sample, the method comprising:

the input step and the selection step executed by using the feature amount selection method according to any one of claims 1 to 6; and
a determination step (S120) of performing the class determination for the unknown sample based on the selected feature amount group, which includes an acquisition step (S122) of acquiring a feature amount value of the selected feature amount group and a class determination step (S124) of performing the class determination based on the acquired feature amount value,
wherein, in the determination step, the class determination for the unknown sample is performed by configuring a multi-class discriminator that uses the selected feature amount group in association with the pairwise coupling.

9. The multi-class classification method according to claim 8,
wherein, in the quantification step (S120), a statistically significant difference in the feature amounts in the learning data set between pairwise-coupled classes is used; and/or wherein, in the quantification step, in a case in which a feature amount of the unknown sample belonging to any of pairwise-coupled classes is given under a threshold value set with reference to the learning data set, a probability of correctly discriminating a class to which the unknown sample belongs by the given feature amount is used; and/or wherein, in the quantification step, a quantification value of the discrimination possibility is a value obtained by performing multiple test correction on a statistical probability value by the number of feature amounts.

10. The multi-class classification method according to claim 8 or 9, further comprising:

a subclass setting step of clustering one or more samples belonging to the classes based on a given feature amount from the learning data set to form a cluster and setting the formed cluster to a subclass in each class;
a second marking step of marking each subclass in each class as second discrimination unneeded class groups that do not need to be discriminated from each other in each class; and
a second exclusion step of excluding the pairwise coupling in the marked second discrimination unneeded class groups from pairwise couplings to be expanded.

11. A non-transitory computer-readable recording medium comprising instructions which, when executed by a computer, cause the computer to carry out the steps of the method according to any of claims 8 to 10.

12. A feature amount selection device (10) that selects a feature amount group to be used for determining which of N (two or more) classes a sample belongs to, the device comprising:

   a first processor (100),
   wherein the first processor performs

   input processing of inputting a learning data set including a known sample group belonging to a given class, which is a target, and a feature amount group of the known sample group, and
   selection processing of selecting a feature amount group needed for class determination for an unknown sample of which a belonging class is unknown, from the feature amount group based on the learning data set, and

   the selection processing includes

   quantification processing of quantifying, by a pairwise coupling that combines two classes among the N classes, a discrimination possibility between the two classes in accordance with each feature amount of the selected feature amount group by using the learning data set, and
   optimization processing of totalizing the quantified discrimination possibilities for all the pairwise couplings and selecting a combination of the feature amount groups for which a result of the totalization is optimized.

   **characterized in that**

   first marking processing of marking a part of the given classes as first discrimination unneeded class groups that do not need to be discriminated from each other, and
   first exclusion processing of excluding the pairwise coupling in the marked first discrimination unneeded class groups from pairwise couplings to be expanded.

13. The feature amount selection device according to claim 12, wherein the first processor (100) further performs

   evaluating processing of evaluating similarity between the feature amounts based on the discrimination possibility for each pairwise coupling of each feature amount, and
   setting processing of setting a priority of the feature amount to be selected, based on an evaluation result of the similarity.

14. A multi-class classification device (10) that determines, in a case in which N is an integer of 2 or more, which of N classes a sample belongs to, from a feature amount of the sample, the device comprising:

   the feature amount selection device according to claim 12; and
   a second processor (100),
   wherein the second processor performs

   the input processing and the selection processing using the feature amount selection device, and
   determination processing of performing the class determination for the unknown sample based on the selected feature amount group, which includes acquisition processing of acquiring a feature amount value of the selected feature amount group and class determination processing of performing the class determination based on the acquired feature amount value, and

   in the determination processing, the class determination for the unknown sample is performed by configuring a multi-class discriminator that uses the selected feature amount group in association with the pairwise coupling.

15. The multi-class classification method of determining according to any one of claims 1 to 10 that is used by the multi-class classification device (10) of claim 14 to determine which of N (two or more) classes a sample belongs.

**Patentansprüche**

1.  Computerimplementiertes Merkmalsmengen-Auswahlverfahren des Auswählens einer Merkmalsmengengruppe, die zum Bestimmen, zu welcher von N (zwei oder mehr) Klassen eine Probe gehört, verwendet wird, wobei das Verfahren umfasst:

    einen Eingabeschritt (S100) des Eingebens eines Lerndatensatzes, der eine bekannte Probengruppe, die zu einer gegebenen Klasse gehört, die ein Ziel ist, und eine Merkmalsmengengruppe der bekannten Probengruppe enthält; und
    einen Auswahlschritt (S110) des Auswählens einer Merkmalsmengengruppe, die für Klassenbestimmung für eine unbekannte Probe, deren zugehörige Klasse unbekannt ist, benötigt wird, aus der Merkmalsmengengruppe auf der Grundlage des Lerndatensatzes,
    wobei der Auswahlschritt (S110) enthält:

    einen Quantifizierungsschritt (S112) des Quantifizierens, durch eine paarweise Kopplung, die zwei Klassen unter den N Klassen kombiniert, einer Diskriminierungsmöglichkeit zwischen den zwei Klassen in Übereinstimmung mit jeder Merkmalsmenge der ausgewählten Merkmalsmengengruppe durch Verwenden des Lerndatensatzes, und
    einen Optimierungsschritt (S114) des Summierens der quantifizierten Diskriminierungsmöglichkeiten für alle paarweisen Kopplungen und des Auswählens einer Kombination der Merkmalsmengengruppen, für die ein Ergebnis der Summierung optimiert ist, **gekennzeichnet durch**
    einen ersten Markierungsschritt des Markierens eines Teils der gegebenen Klassen als erste Diskriminierung nicht benötigende Klassengruppen, die nicht voneinander diskriminiert werden müssen, und
    einen ersten Ausschlussschritt des Ausschlusses der paarweisen Kopplung in den markierten ersten Diskriminierung nicht benötigenden Klassengruppen, aus paarweisen Kopplungen, die zu erweitern sind.

2.  Merkmalsmengen-Auswahlverfahren nach Anspruch 1,
    wobei der Auswahlschritt ferner enthält:

    einen Ähnlichkeitsbewertungsschritt des Bewertens von Ähnlichkeit zwischen den Merkmalsmengen auf der Grundlage der Diskriminierungsmöglichkeit für jede paarweise Kopplung jeder Merkmalsmenge, und
    einen Prioritätseinstellschritt des Einstellens einer Priorität der auszuwählenden Merkmalsmenge auf der Grundlage eines Bewertungsergebnisses der Ähnlichkeit.

3.  Merkmalsmengen-Auswahlverfahren nach Anspruch 2,
    wobei die Ähnlichkeit eine Überlappungsbeziehung und/oder eine Einschlussbeziehung der Diskriminierungsmöglichkeit für jede paarweise Kopplung ist.

4.  Merkmalsmengen-Auswahlverfahren nach Anspruch 2 oder 3,
    wobei die Ähnlichkeit eine Distanz zwischen Vektoren von Diskriminierungsmöglichkeit für jede paarweise Kopplung oder ein Metrikwert in Übereinstimmung mit der Distanz ist.

5.  Merkmalsmengen-Auswahlverfahren nach einem der Ansprüche 1 bis 4, ferner umfassend:

    einen ausgewählten Zahlen-Eingabeschritt des Eingebens einer ausgewählten Zahl M der Merkmalsmengen bei dem Auswahlschritt,
    wobei die Optimierung Maximieren eines Minimalwerts eines summierten Werts bei allen paarweisen Kopplungen in Übereinstimmung mit M ausgewählten Merkmalsmengen ist.

6.  Merkmalsmengen-Auswahlverfahren nach einem der Ansprüche 1 bis 5,
    wobei der Optimierungsschritt enthält:

    einen Wichtigkeitseingabeschritt des Eingebens von Wichtigkeit der Klasse oder paarweiser Diskriminierung, und
    einen Gewichtungsschritt des Durchführens von Gewichtung auf der Grundlage der Wichtigkeit in einem Fall der Summierung.

7.  Nicht flüchtiges computerlesbares Aufzeichnungsmedium, umfassend Anweisungen, die, wenn sie von einem Computer ausgeführt werden, den Computer veranlassen, die Schritte des Verfahrens nach einem der Ansprüche 1 bis 6 durchzuführen.

8. Mehrklassen-Klassifizierungsverfahren des Bestimmens, in einem Fall, in dem N eine ganze Zahl von 2 oder mehr ist, zu welcher von N Klassen eine Probe gehört, aus einer Merkmalsmenge der Probe, wobei das Verfahren umfasst:

den Eingabeschritt und den Auswahlschritt, die durch Verwenden des Merkmalsmengen-Auswahlverfahrens nach einem der Ansprüche 1 bis 6 ausgeführt werden; und

einen Bestimmungsschritt (S120) des Durchführens der Klassenbestimmung für die unbekannte Probe auf der Grundlage der ausgewählten Merkmalsmengengruppe, der einen Erfassungsschritt (S122) des Erfassens eines Merkmalsmengenwerts von der ausgewählten Merkmalsmengengruppe und einen Klassenbestimmungsschritt (S124) des Durchführens der Klassenbestimmung auf der Grundlage des erfassten Merkmalsmengenwerts umfasst,

wobei bei dem Bestimmungsschritt die Klassenbestimmung für die unbekannte Probe durch Konfigurieren eines Mehrklassendiskriminators, der die ausgewählte Merkmalsmengengruppe in Verbindung mit der paarweisen Kopplung verwendet, durchgeführt wird.

9. Mehrklassen-Klassifizierungsverfahren nach Anspruch 8,

wobei bei dem Quantifizierungsschritt (S120) ein statistisch signifikanter Unterschied der Merkmalsmengen in dem Lerndatensatz zwischen paarweise gekoppelten Klassen verwendet wird; und/oder wobei bei dem Quantifizierungsschritt in einem Fall, in dem eine Merkmalsmenge der unbekannten Probe, die zu einer beliebigen von paarweise gekoppelten Klassen gehört, unter einem Schwellenwert, der mit Bezug auf den Lerndatensatz eingestellt ist, gegeben ist, eine Wahrscheinlichkeit des korrekten Diskriminierens einer Klasse, zu der die unbekannte Probe gehört, durch die gegebene Merkmalsmenge verwendet wird; und/oder wobei bei dem Quantifizierungsschritt ein Quantifizierungswert der Diskriminierungsmöglichkeit ein Wert ist, der durch Durchführen von Korrektur an Mehrfachtests an einem statistischen Wahrscheinlichkeitswert durch die Anzahl von Merkmalsmengen erhalten wird.

10. Mehrklassen-Klassifizierungsverfahren nach Anspruch 8 oder 9, ferner umfassend:

einen Unterklassen-Einstellschritt des Clusterns einer oder mehrerer Proben, die zu den Klassen gehören, auf der Grundlage einer gegebenen Merkmalsmenge aus dem Lerndatensatz, um einen Cluster zu bilden, und des Einstellens des gebildeten Clusters zu einer Unterklasse in jeder Klasse;

einen zweiten Markierungsschritt des Markierens jeder Unterklasse in jeder Klasse als zweite Diskriminierung nicht benötigende Klassengruppen, die nicht in jeder Klasse voneinander diskriminiert werden müssen; und

einen zweiten Ausschlussschritt des Ausschlusses der paarweisen Kopplung in den markierten zweiten Diskriminierung nicht benötigenden Klassengruppen, aus paarweisen Kopplungen, die zu erweitern sind.

11. Nicht flüchtiges computerlesbares Aufzeichnungsmedium, umfassend Anweisungen, die, wenn sie von einem Computer ausgeführt werden, den Computer veranlassen, die Schritte des Verfahrens nach einem der Ansprüche 8 bis 10 durchzuführen.

12. Merkmalsmengen-Auswahlvorrichtung (10), die eine Merkmalsmengengruppe auswählt, die zum Bestimmen, zu welcher von N (zwei oder mehr) Klassen eine Probe gehört, verwendet wird, wobei die Vorrichtung umfasst:

einen ersten Prozessor (100),
wobei der erste Prozessor durchführt:

Eingabeverarbeitung des Eingebens eines Lerndatensatzes, der eine bekannte Probengruppe, die zu einer gegebenen Klasse gehört, die ein Ziel ist, und eine Merkmalsmengengruppe der bekannten Probengruppe enthält, und

Auswahlverarbeitung des Auswählens einer Merkmalsmengengruppe, die für Klassenbestimmung für eine unbekannte Probe, deren zugehörige Klasse unbekannt ist, benötigt wird, aus der Merkmalsmengengruppe auf der Grundlage des Lerndatensatzes, und

die Auswahlverarbeitung enthält:

Quantifizierungsverarbeitung des Quantifizierens, durch eine paarweise Kopplung, die zwei Klassen unter den N Klassen kombiniert, einer Diskriminierungsmöglichkeit zwischen den zwei Klassen in Übereinstimmung mit jeder Merkmalsmenge der ausgewählten Merkmalsmengengruppe durch Verwenden des Lerndatensatzes, und

Optimierungsverarbeitung des Summierens der quantifizierten Diskriminierungsmöglichkeiten für alle paarweisen Kopplungen und des Auswählens einer Kombination der Merkmalsmengengruppe, für die ein Ergebnis der Summierung optimiert ist,

**dadurch gekennzeichnet, dass**

erste Markierungsverarbeitung des Markierens eines Teils der gegebenen Klassen als erste Diskriminierung nicht benötigende Klassengruppen, die nicht voneinander diskriminiert werden müssen, und erste Ausschlussverarbeitung des Ausschlusses der paarweisen Kopplung in den markierten ersten Diskriminierung nicht benötigenden Klassengruppen aus paarweisen Kopplungen, die zu erweitern sind.

**13.** Merkmalsmengen-Auswahlvorrichtung nach Anspruch 12, wobei der erste Prozessor (100) ferner durchführt:

Bewertungsverarbeitung des Bewertens von Ähnlichkeit zwischen den Merkmalsmengen auf der Grundlage der Diskriminierungsmöglichkeit für jede paarweise Kopplung jeder Merkmalsmenge, und Einstellungsverarbeitung des Einstellens einer Priorität der auszuwählenden Merkmalsmenge auf der Grundlage eines Bewertungsergebnisses der Ähnlichkeit.

**14.** Mehrklassen-Klassifizierungsvorrichtung (10), die in einem Fall, in dem N eine ganze Zahl von 2 oder mehr ist, bestimmt, zu welcher von N Klassen eine Probe gehört, aus einer Merkmalsmenge der Probe, wobei die Vorrichtung umfasst:

die Merkmalsmengen-Auswahlvorrichtung nach Anspruch 12, und einen zweiten Prozessor (100), wobei der zweite Prozessor durchführt:

die Eingangsverarbeitung und die Auswahlverarbeitung unter Verwendung der Merkmalsmengen-Auswahlvorrichtung, und

Bestimmungsverarbeitung des Verarbeitens der Klassenbestimmung für die unbekannte Probe auf der Grundlage der ausgewählten Merkmalsmengengruppe, die Erfassungsverarbeitung des Erfassens eines Merkmalsmengenwerts der ausgewählten Merkmalsmengengruppe und Klassenbestimmungsverarbeitung des Durchführen der Klassenbestimmung auf der Grundlage des erfassten Merkmalsmengenwerts enthält, und

bei der Bestimmungsverarbeitung, die Klassenbestimmung für die unbekannte Probe, die durch Konfigurieren eines Mehrklassen-Diskriminators, der die ausgewählte Merkmalsmengengruppe in Verbindung mit der paarweisen Kopplung verwendet, durchgeführt wird.

**15.** Mehrklassen-Klassifizierungsverfahren des Bestimmens nach einem der Ansprüche 1 bis 10, das von der Mehrklassen-Klassifizierungsvorrichtung (10) nach Anspruch 14 verwendet wird, um zu bestimmen, zu welcher von N (zwei oder mehr) Klassen eine Probe gehört.

## Revendications

**1.** Procédé de sélection de quantité de caractéristiques mise en œuvre par ordinateur pour sélectionner un groupe de quantité de caractéristiques à utiliser pour déterminer à laquelle des N (deux ou plusieurs) classes un échantillon appartient, le procédé comprenant :

une étape d'entrée (S100) consistant à entrer un ensemble de données d'apprentissage incluant un groupe d'échantillons connu appartenant à une classe donnée, qui est une cible, et un groupe de quantité de caractéristiques du groupe d'échantillons connu ; et

une étape de sélection (S110) consistant à sélectionner un groupe de quantité de caractéristiques nécessaire à la détermination de classe pour un échantillon inconnu dont une classe d'appartenance est inconnue, à partir du groupe de quantité de caractéristiques sur la base de l'ensemble de données d'apprentissage,

dans lequel l'étape de sélection (S110) inclut

une étape de quantification (S112) consistant à quantifier, par un couplage par paires qui combine deux classes parmi les N classes, une possibilité de discrimination entre les deux classes conformément à chaque quantité de caractéristiques du groupe de quantité de caractéristiques sélectionné en utilisant l'ensemble de données d'apprentissage, et

une étape d'optimisation (S114) consistant à totaliser les possibilités de discrimination quantifiées pour tous les couplages par paires et à sélectionner une combinaison des groupes de quantité de caractéristiques pour lesquels un résultat de la totalisation est optimisé, **caractérisée par**

une première étape de marquage consistant à marquer une partie des classes données comme premiers groupes de classes non nécessaires à la discrimination qui n'ont pas besoin d'être discriminés les uns des autres, et

une première étape d'exclusion consistant à exclure la couplage par paires dans les premiers groupes de classes non nécessaires à la discrimination marqués des couplages par paires à étendre.

2. Procédé de sélection de quantité de caractéristiques selon la revendication 1,

dans lequel l'étape de sélection inclut en outre
une étape d'évaluation de similarité consistant à évaluer la similarité entre les quantités de caractéristiques sur la base de la possibilité de discrimination pour chaque couplage par paires de chaque quantité de caractéristiques, et

une étape de définition de priorité consistant à définir une priorité de la quantité de caractéristiques à sélectionner, sur la base d'un résultat d'évaluation de la similarité.

3. Procédé de sélection de quantité de caractéristiques selon la revendication 2,
dans lequel la similarité est une relation de chevauchement et/ou une relation d'inclusion de la possibilité de discrimination pour chaque couplage par paires.

4. Procédé de sélection de quantité de caractéristiques selon la revendication 2 ou la revendication 3,
dans lequel la similarité est une distance entre des vecteurs de possibilité de discrimination pour chaque couplage par paires ou une valeur métrique conformément à la distance.

5. Procédé de sélection de quantité de caractéristiques selon l'une quelconque des revendications 1 à 4, comprenant en outre :

une étape d'entrée de nombre sélectionné consistant à entrer un nombre sélectionné M des quantités de caractéristique dans l'étape de sélection,
dans lequel l'optimisation consiste à maximiser une valeur minimale d'une valeur totalisée dans tous les couplages par paires conformément à M quantités de caractéristiques sélectionnées.

6. Procédé de sélection de quantité de caractéristiques selon l'une quelconque des revendications 1 à 5,

dans lequel l'étape d'optimisation inclut
une étape d'entrée d'importance consistant à entrer l'importance de la classe ou de la discrimination par paires, et
une étape de pondération consistant à effectuer une pondération sur la base de l'importance dans un cas de la totalisation.

7. Support d'enregistrement non transitoire lisible par ordinateur comprenant des instructions qui, lorsqu'elles sont exécutées par un ordinateur, amènent l'ordinateur à effectuer les étapes du procédé selon l'une quelconque des revendications 1 à 6.

8. Procédé de classification multi-classes de déterminer, dans un cas où N est un entier de 2 ou plus, à laquelle des N classes un échantillon appartient, à partir d'une quantité de caractéristiques de l'échantillon, le procédé comprenant :

l'étape d'entrée et l'étape de sélection exécutées en utilisant le procédé de sélection de quantité de caractéristiques selon l'une quelconque des revendications 1 à 6 ; et
une étape de détermination (S120) consistant à effectuer la détermination de classe pour l'échantillon inconnu sur la base du groupe de quantité de caractéristiques sélectionné, qui inclut une étape d'acquisition (S122) consistant à acquérir une valeur de quantité de caractéristiques du groupe de quantité de caractéristiques sélectionné et
une étape de détermination de classe (S124) consistant à effectuer la détermination de classe sur la base de la valeur de quantité de caractéristiques acquise,
dans lequel, dans l'étape de détermination, la détermination de classe pour l'échantillon inconnu est effectuée en configurant un discriminateur multi-classes qui utilise le groupe de quantité de caractéristiques sélectionné en association avec le couplage par paires.

**9.** Procédé de classification multi-classes selon la revendication 8,

dans lequel, dans l'étape de quantification (S120), une différence statistiquement significative dans les quantités de caractéristiques dans l'ensemble de données d'apprentissage entre des classes couplées par paires est utilisée ; et/ou dans lequel, dans l'étape de quantification, dans un cas où une quantité de caractéristiques de l'échantillon inconnu appartenant à l'une quelconque des classes couplées par paires est donnée sous une valeur seuil définie en référence à l'ensemble de données d'apprentissage, une probabilité de discriminer correctement une classe à laquelle l'échantillon inconnu appartient par la quantité de caractéristiques donnée est utilisée ; et/ou dans lequel, dans l'étape de quantification, une valeur de quantification de la possibilité de discrimination est une valeur obtenue en effectuant une correction de test multiple sur une valeur de probabilité statistique par le nombre de quantités de caractéristiques.

**10.** Procédé de classification multi-classes selon la revendication 8 ou la revendication 9, comprenant en outre :

une étape de définition de sous-classe consistant à regrouper un ou plusieurs échantillons appartenant aux classes sur la base d'une quantité de caractéristiques donnée à partir de l'ensemble de données d'apprentissage pour former un cluster et à définir le cluster formé comme sous-classe dans chaque classe ; une deuxième étape de marquage consistant à marquer chaque sous-classe dans chaque classe comme deuxièmes groupes de classes non nécessaires à la discrimination qui n'ont pas besoin d'être discriminés les uns des autres dans chaque classe ; et une deuxième étape d'exclusion consistant à exclure le couplage par paires dans les deuxièmes groupes de classes non nécessaires à la discrimination marqués à partir des couplages par paires à étendre.

**11.** Support d'enregistrement non transitoire lisible par ordinateur comprenant des instructions qui, lorsqu'elles sont exécutées par un ordinateur, amènent l'ordinateur à effectuer les étapes du procédé selon l'une quelconque des revendications 8 à 10.

**12.** Dispositif de sélection de quantité de caractéristiques (10) qui sélectionne un groupe de quantité de caractéristiques à utiliser pour déterminer à laquelle des N (deux ou plusieurs) classes un échantillon appartient, le dispositif comprenant :

un premier processeur (100), dans lequel le premier processeur effectue un traitement d'entrée consistant à entrer un ensemble de données d'apprentissage incluant un groupe d'échantillons connu appartenant à une classe donnée, qui est une cible, et un groupe de quantité de caractéristiques du groupe d'échantillons connu, et un traitement de sélection consistant à sélectionner un groupe de quantité de caractéristiques nécessaire à la détermination de classe pour un échantillon inconnu dont une classe d'appartenance est inconnue, à partir du groupe de quantité de caractéristiques sur la base de l'ensemble de données d'apprentissage, et le traitement de sélection inclut un traitement de quantification consistant à quantifier, par un couplage par paires qui combine deux classes parmi les N classes, une possibilité de discrimination entre les deux classes conformément à chaque quantité de caractéristiques du groupe de quantité de caractéristiques sélectionné en utilisant l'ensemble de données d'apprentissage, et un traitement d'optimisation consistant à totaliser les possibilités de discrimination quantifiées pour tous les couplages par paires et sélectionner une combinaison des groupes de quantité de caractéristiques pour lesquels un résultat de la totalisation est optimisé, **caractérisé en ce que** un première traitement de marquage consistant à marquer une partie des classes données comme premiers groupes de classes non nécessaires à la discrimination qui n'ont pas besoin d'être discriminés les uns des autres, et un première traitement d'exclusion consistant à exclure le couplage par paires dans les premiers groupes de classes non nécessaires à la discrimination marqués des couplages par paires à étendre.

**13.** Dispositif de sélection de quantité de caractéristiques selon la revendication 12, dans lequel le premier processeur (100) effectue en outre

un traitement d'évaluation consistant à évaluer la similarité entre les quantités de caractéristiques sur la base de la possibilité de discrimination pour chaque couplage par paires de chaque quantité de caractéristiques, et
un traitement de définition consistant à définir une priorité de la quantité de caractéristiques à sélectionner, sur la base d'un résultat d'évaluation de la similarité.

14. Dispositif de classification multi-classes (10) qui détermine, dans un cas où N est un entier de 2 ou plus, à laquelle des N classes un échantillon appartient, à partir d'une quantité de caractéristiques de l'échantillon, le dispositif comprenant :

le dispositif de sélection de quantité de caractéristiques selon la revendication 12 ; et
un deuxième processeur (100),
dans lequel le deuxième processeur effectue
le traitement d'entrée et le traitement de sélection à l'aide du dispositif de sélection de quantité de caractéristiques, et
un traitement de détermination consistant à effectuer la détermination de classe pour l'échantillon inconnu sur la base du groupe de quantité de caractéristiques sélectionné,
qui inclut un traitement d'acquisition consistant à acquérir une valeur de quantité de caractéristiques du groupe de quantité de caractéristiques sélectionné et un traitement de détermination consistant à effectuer la détermination de classe sur la base de la valeur de quantité de caractéristiques acquise, et
dans le traitement de détermination, la détermination de classe pour l'échantillon inconnu est effectuée en configurant un discriminateur multi-classes qui utilise le groupe de quantité de caractéristiques sélectionné en association avec le couplage par paires.

15. Procédé de classification multi-classes de déterminer selon l'une quelconque des revendications 1 à 10, qui est utilisé par le dispositif de classification multi -classes (10) de la revendication 14 pour déterminer à laquelle des N (deux ou plusieurs) classes un échantillon appartient.

# FIG. 1

STEP1 FEATURE SELECTION

STEP2 MULTI-CLASS CLASSIFICATION

EP 4 105 881 B1

# FIG. 2

# FIG. 3

100

| INPUT PROCESSING UNIT | — 102 |
|---|---|

SELECTION PROCESSING UNIT — 104

| QUANTIFICATION PROCESSING UNIT | — 106 |
|---|---|

| OPTIMIZATION PROCESSING UNIT | — 108 |
|---|---|

DETERMINATION PROCESSING UNIT — 110

| ACQUISITION PROCESSING UNIT | — 112 |
|---|---|

| CLASS DETERMINATION PROCESSING UNIT | — 114 |
|---|---|

| OUTPUT PROCESSING UNIT | — 115 |
|---|---|

| CPU | — 116 |
|---|---|

| ROM | — 118 |
|---|---|

| RAM | — 120 |
|---|---|

FIG. 4

```
┌────────────────────────────────────┐
│             INPUT STEP             │──── S100
└────────────────────────────────────┘
                  │
                  ▼
┌────────────────────────────────────┐
│           SELECTION STEP           │──── S110
│  ┌──────────────────────────────┐  │
│  │     QUANTIFICATION STEP      │──┼── S112
│  └──────────────────────────────┘  │
│                │                   │
│                ▼                   │
│  ┌──────────────────────────────┐  │
│  │      OPTIMIZATION STEP       │──┼── S114
│  └──────────────────────────────┘  │
└────────────────────────────────────┘
                  │
                  ▼
┌────────────────────────────────────┐
│         DETERMINATION STEP         │──── S120
│  ┌──────────────────────────────┐  │
│  │      ACQUISITION STEP        │──┼── S122
│  └──────────────────────────────┘  │
│                │                   │
│                ▼                   │
│  ┌──────────────────────────────┐  │
│  │   CLASS DETERMINATION STEP   │──┼── S124
│  └──────────────────────────────┘  │
└────────────────────────────────────┘
```

## FIG. 5A

## FIG. 5B

## FIG. 6A

CLASS

|  | | A | B | C |
|---|---|---|---|---|
| **FEATURE AMOUNT** | #1 | 1 | 1 | 1 |
| | #2 | 0 | 1 | 1 |
| | #3 | 1 | 0 | 0 |
| | #4 | 1 | 1 | 0 |
| | #5 | 0 | 0 | 0 |

## FIG. 6B

PAIRWISE
(CLASS BINARY
RELATIONSHIP)

| A | B | C |
|---|---|---|

| A | B | |
|---|---|---|

| A | | C |
|---|---|---|

| | B | C |
|---|---|---|

## FIG. 6C

|  | {A,B} | {A,C} | {B,C} |
|---|---|---|---|
| #1 | 0 | 0 | 0 |
| #2 | 1 | 1 | 0 |
| #3 | 1 | 1 | 0 |
| #4 | 0 | 1 | 1 |
| #5 | 0 | 0 | 0 |

FIG. 7A

FIG. 7B

DISCRIMINATION SWITCH VALUE = 1
* STATE VALUE DEPENDS ON MEASUREMENT VALUE

CLASS A SAMPLE DISTRIBUTION

SET THRESHOLD VALUE

HOLD

A

B

CLASS B SAMPLE DISTRIBUTION

PROBABILITY DENSITY

VALUE OF FEATURE AMOUNT

DISCRIMINATION SWITCH VALUE = 0
* STATE VALUE IS ALWAYS 0

CLASS A SAMPLE DISTRIBUTION

CLASS B SAMPLE DISTRIBUTION

PROBABILITY DENSITY

VALUE OF FEATURE AMOUNT

EP 4 105 881 B1

EP 4 105 881 B1

# FIG. 8

CLASS GROUP THAT NEEDS
TO BE DISCRIMINATED

CLASS GROUP THAT NEEDS
TO BE DISCRIMINATED AS NOT T
BUT DOES NOT NEED TO BE
DISCRIMINATED BETWEEN CLASSES

| T1 | T2 | . . . | Tm | N1 | N2 | . . . | Nn |

$N \times N$

NOT EXPANDED

EXAMPLE: CANCER
TISSUE

EXAMPLE: NORMAL TISSUE

PAIRWISE EXPANSION

$T \times T$

$T \times N$

| T1 | T2 | | |

| T1 | | N1 | |

| | T2 | N1 | |

| T1 | | T3 | |

| T1 | | | N2 |

| | T2 | | N2 |

. . .

. . .

# FIG. 9A

CLASS B
BELONGING SAMPLE
DISTANCE SPACE
DISTRIBUTION

TYPE Z

TYPE X

TYPE Y

# FIG. 9B

FEATURE AMOUNT i

SUBCLASS X ?

SUBCLASS Y ?

CLASS A

CLASS B

## FIG. 10A

|  | {A,B} |
|---|---|
| #1 | +1 |
| #2 | 0 |
| ... |  |
| SUBTOTAL | 24 |

|  | {A,C} |
|---|---|
| #3 | −1 |
| #4 | +1 |
| ... |  |
| SUBTOTAL | −2 |

|  | {A,...} |
|---|---|
| #... | +1 |
|  | 0 |
| ... |  |
| SUBTOTAL | 13 |

## FIG. 10B

| {A,*} | A |
|---|---|
| {A,B} | +1 |
| {A,C} | −1 |
| {A,...} | ... |
| TOTAL | 7 |

. . .

| {D,*} | D |
|---|---|
| {D,A} | +1 |
| {D,B} | 0 |
| {D,...} | ... |
| TOTAL | 10 |

## FIG. 10C

| CLASS | SCORE |
|---|---|
| $D_{(1)}$ | 10 |
| $N_{(2)}$ | 8 |
| $A_{(3)}$ | 7 |
| ... |  |

# FIG. 11

WINNER OF N·A
AND BRUTE FORCE MATCH
FIRST RANK D ARE
COMPARED DIRECTLY

BRUTE FORCE MATCH
SECOND RANK N
AND BRUTE FORCE MATCH
THIRD RANK A
ARE COMPARED DIRECTLY

$D_{(1)}$

$N_{(2)}$

$A_{(3)}$

BRUTE FORCE
MATCH
FIRST RANK

BRUTE FORCE
MATCH
SECOND RANK

BRUTE FORCE
MATCH
THIRD RANK

# FIG. 12

| | CLASS | SUBTYPE | SAMPLE SIZE | | TOTAL |
|---|---|---|---|---|---|
| | TISSUE | CANCER CLASSIFICATION | CANCER | NORMAL | |
| 1a | LARGE INTESTINE | COLONIC GLAND CANCER | 312 | 38 | 350 |
| 1b | " | RECTAL GLAND CANCER | 98 | 7 | 105 |
| 2 | STOMACH | STOMACH CANCER | 395 | 2 | 397 |
| 3a | LUNG | LUNG CANCER | 473 | 32 | 505 |
| 3b | " | LUNG EPITHELIUM SQUAMOUS CANCER | 192 | 42 | 234 |
| 4 | BREAST | BREAST CANCER | 792 | 97 | 889 |
| 5 | PROSTATE | PROSTATE CANCER | 502 | 50 | 552 |
| 6 | PANCREAS | PANCREATIC CANCER | 184 | 10 | 194 |
| 7 | LIVER | LIVER CANCER | 377 | 50 | 427 |
| 8 | UTERUS | CERVICAL CANCER | 307 | 3 | 310 |
| 9a | KIDNEY | *CLEAR CELL CANCER | – | 160 | 160 |
| 9b | " | *PAPILLARY RENAL CELL CANCER | – | 45 | 45 |
| 10 | THYROID GLAND | – | – | 56 | 56 |
| 11 | HEAD AND NECK | – | – | 50 | 50 |
| 12 | UTERINE BODY | – | – | 46 | 46 |
| 13 | BLADDER/UROTHELIUM | – | – | 21 | 21 |
| 14 | ESOPHAGUS | – | – | 16 | 16 |
| 15 | BILE DUCT | – | – | 9 | 9 |
| 16 | BONE/SOFT TISSUE | – | – | 4 | 4 |
| 17 | SUPRARENAL GLAND | – | – | 3 | 3 |
| 18 | THYMUS | – | – | 2 | 2 |
| 19 | SKIN | – | – | 2 | 2 |
| 20 | BRAIN | – | – | 1 | 1 |
| 21 | BLOOD | – | – | 732 | 732 |
| | TOTAL | | 3,632 | 1,478 | 5,110 |

## FIG. 13

| METHOD | ACCURACY ITEM | AVERAGE VALUE | LARGE INTESTINE CANCER | STOMACH CANCER | LUNG CANCER | BREAST CANCER | PROSTATE CANCER | PANCREATIC CANCER | LIVER CANCER | CERVICAL CANCER |
|---|---|---|---|---|---|---|---|---|---|---|
| PRESENT INVENTION | F-NUMBER | 0.953 | 0.976 | 0.938 | 0.965 | 0.969 | 0.974 | 0.883 | 0.965 | 0.957 |
| | SENSITIVITY | 0.952 | 0.980 | 0.929 | 0.946 | 0.962 | 0.960 | 0.946 | 0.941 | 0.948 |
| | GOODNESS OF FIT | 0.957 | 0.971 | 0.948 | 0.984 | 0.977 | 0.988 | 0.829 | 0.989 | 0.967 |
| RELATED-ART METHOD | F-NUMBER | 0.809 | 0.835 | 0.650 | 0.756 | 0.859 | 0.970 | 0.604 | 0.906 | 0.889 |
| | SENSITIVITY | 0.808 | 0.833 | 0.594 | 0.910 | 0.886 | 0.964 | 0.522 | 0.872 | 0.883 |
| | GOODNESS OF FIT | 0.821 | 0.837 | 0.718 | 0.646 | 0.834 | 0.976 | 0.716 | 0.943 | 0.895 |

EP 4 105 881 B1

## FIG. 14

| METHOD | DATA SET | AVERAGE VALUE | LARGE INTESTINE CANCER | STOMACH CANCER | LUNG CANCER | BREAST CANCER | PROSTATE CANCER | PANCREATIC CANCER | LIVER CANCER | CERVICAL CANCER |
|---|---|---|---|---|---|---|---|---|---|---|
| PRESENT INVENTION | DIFFERENCE | 0.008 | 0.012 | 0.024 | -0.016 | 0.005 | 0.006 | 0.017 | 0.011 | 0.007 |
| | LEARNING | 0.962 | 0.988 | 0.962 | 0.948 | 0.975 | 0.980 | 0.901 | 0.976 | 0.965 |
| | TEST | 0.953 | 0.976 | 0.938 | 0.965 | 0.969 | 0.974 | 0.883 | 0.965 | 0.957 |
| RELATED-ART METHOD | DIFFERENCE | 0.185 | 0.160 | 0.345 | 0.222 | 0.123 | 0.030 | 0.396 | 0.091 | 0.111 |
| | LEARNING | 0.993 | 0.995 | 0.995 | 0.978 | 0.982 | 1.000 | 1.000 | 0.997 | 1.000 |
| | TEST | 0.809 | 0.835 | 0.650 | 0.756 | 0.859 | 0.970 | 0.604 | 0.906 | 0.889 |

EP 4 105 881 B1

FIG. 15

EP 4 105 881 B1

# FIG. 16

**(a)**

CLASSIFICATION RESULT CLASS AND HIGHER RANK CAN BE CONFIRMED

### DETERMINATION SCORE / FINAL RESULT TABLE

| Sample | CANCER TISSUE_247 |
|---|---|
| Estimated Class | CANCER TISSUE 1 |
| Score Ranking [1] | CANCER TISSUE 1 (79) |
| Score Ranking [2] | CANCER TISSUE 3 (76) |
| CANCER TISSUE 1 | 79 |
| CANCER TISSUE 3 | 76 |
| CANCER TISSUE 4 | -80 |
| CANCER TISSUE 5 | 46 |
| NORMAL TISSUE 3 | 17 |
| NORMAL TISSUE 2 | 23 |
| NORMAL TISSUE 1 | -7 |
| <CANCER TISSUE 1 \|CANCER TISSUE 3> | 9 |
| <CANCER TISSUE 1 \|CANCER TISSUE 4> | 151 |
| <CANCER TISSUE 1 \|CANCER TISSUE 5> | 32 |

SIMILAR SCORE WITH EACH CLASS AND PAIR SCORE CAN BE CONFIRMED

REASON OF SELECTING MARKER WHICH IS BASIS FOR PAIR DETERMINATION CAN BE CONFIRMED

*ACTUAL DETERMINATION TRANSITION IN TEST DATA IS EXTRACTED

**(b)**

SELECTED MARKER LIST

| Marker | <CANCER TISSUE 1 \|CANCER TISSUE 2> | <CANCER TISSUE 1 \|CANCER TISSUE 3> | <CANCER TISSUE 1 \|CANCER TISSUE 4> | <CANCER TISSUE 1 \|CANCER TISSUE 5> |
|---|---|---|---|---|
| MARKER 1 | 1 | 1 | 1 | 1 |
| MARKER 2 | 0 | 0 | 0 | 1 |
| MARKER 3 | 0 | 1 | 1 | 1 |
| MARKER 4 | 0 | 1 | 1 | 1 |
| MARKER 5 | 0 | 0 | 1 | 0 |
| MARKER 6 | 0 | 0 | 1 | 0 |
| MARKER 7 | 0 | 0 | 1 | 0 |
| MARKER 8 | 0 | 0 | 0 | 0 |
| MARKER 9 | 0 | 1 | 1 | 0 |
| MARKER 10 | 0 | 1 | 0 | 1 |
| MARKER 11 | 0 | 1 | 0 | 0 |
| MARKER 12 | 0 | 0 | 1 | 0 |
| MARKER 13 | 0 | 0 | 0 | 1 |
| MARKER 14 | 1 | 0 | 1 | 0 |
| MARKER 15 | 0 | 0 | 0 | 1 |
| MARKER 16 | 0 | 0 | 0 | 1 |
| MARKER 17 | 0 | 0 | 1 | 0 |
| MARKER 18 | 0 | 0 | 1 | 0 |
| MARKER 19 | 0 | 0 | 0 | 1 |
| MARKER 20 | 0 | 0 | 0 | 0 |
| MARKER 21 | 0 | 0 | 1 | 1 |
| MARKER 22 | 0 | 0 | 0 | 0 |
| MARKER 23 | 0 | 0 | 1 | 0 |
| MARKER 24 | 0 | 0 | 0 | 1 |
| MARKER 25 | 0 | 0 | 0 | 1 |
| MARKER 26 | 0 | 0 | 1 | 0 |
| MARKER 27 | 0 | 0 | 1 | 1 |
| MARKER 28 | 0 | 0 | 0 | 0 |
| MARKER 29 | 0 | 0 | 1 | 0 |
| MARKER 30 | 0 | 0 | 1 | 1 |
| MARKER 31 | 0 | 0 | 1 | 1 |
| MARKER 32 | 0 | 0 | 1 | 0 |
| MARKER 33 | 0 | 0 | 0 | 0 |

## FIG. 17

## FIG. 18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012505453 A

**Non-patent literature cited in the description**

- **HYEON JI et al.** Feature selection for multi-class classification using pairwise class discriminatory measure and covering concept. *ELECTRONICS LETTERS*, 16 March 2000, vol. 36 (6), 524-525